(19) 

**Europäisches Patentamt**
**European Patent Office**
**Office européen des brevets**

(11) **EP 4 400 039 A1**

(12) **EUROPEAN PATENT APPLICATION**

(43) Date of publication:
**17.07.2024 Bulletin 2024/29**

(21) Application number: **23151041.3**

(22) Date of filing: **11.01.2023**

(51) International Patent Classification (IPC):
**A61B 5/022** *(2006.01)* **A61B 5/00** *(2006.01)*

(52) Cooperative Patent Classification (CPC):
**A61B 5/02225; A61B 5/7246**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Koninklijke Philips N.V.**
**5656 AG Eindhoven (NL)**

(72) Inventors:
• **MUEHLSTEFF, Jens**
  **Eindhoven (NL)**

• **SCHMITT, Lars**
  **Eindhoven (NL)**
• **DAVIDOIU, Valentina-Geta**
  **5656AG Eindhoven (NL)**
• **WIJSHOFF, Ralph Wilhelm Christianus Gemma Rosa**
  **Eindhoven (NL)**

(74) Representative: **Philips Intellectual Property & Standards**
**High Tech Campus 52**
**5656 AG Eindhoven (NL)**

(54) **HEMODYNAMIC PARAMETER MEASUREMENT**

(57) A method for deriving one or more hemodynamic parameters, including one or more measures of blood pressure, based on acquiring a series of sample pulse wave signals at a series of applied pressures using a tissue pressure sensor incorporated between a user's body and a pressure applicator. The hemodynamic parameters are derived based on identifying characteristic points in a derived plot indicative of a change in pulse waveform morphology as a function of applied pressure.

**EP 4 400 039 A1**

50

| Obtain tissue pressure signals for series of applied pressures | 52 |

↓

| Extract candidate pulse wave signals | 54 |

↓

| Plot change in waveform morphology as function of applied pressure | 56 |

↓

| Identify characteristic points | 58 |

↓

| Map characteristic points to BP values | 60 |

↓

| Output BP values | 62 |

FIG. 13

**Description**

FIELD OF THE INVENTION

The present invention relates to a method for measuring hemodynamic parameters such as blood pressure.

BACKGROUND OF THE INVENTION

Monitoring of hemodynamic parameters plays an important role in patient monitoring, particularly for critically ill patients. Hemodynamic monitoring allows for monitoring tissue perfusion and oxygen delivery while maintaining adequate mean arterial blood pressure for a patient. In most clinical practice, such monitoring is only available in more intensive care environments due to the invasive nature of currently available technologies.

In recent years, advances have been made in developing non-invasive hemodynamic monitoring techniques.

One area of interest is the possibility to use measurement data acquired using an inflatable measurement cuff (i.e. a sphygmomanometer system), typically used for blood pressure measurement, to also non-invasively determine estimates for central hemodynamic parameters such as cardiac output (CO), stroke volume (SV), stroke volume variation (SVV), in addition to various measures of arterial blood pressure, such as mean arterial pressure (MAP), systolic blood pressure (SBP) and diastolic blood pressure (DBP).

One example system developed for non-invasive hemodynamic monitoring is the device detailed in document EP2953528 A1.

This describes a blood pressure measuring system which includes a separate sensor for measuring a pressure between the user's body part (e.g. arm) and the cuff. This will be referred to as a tissue pressure sensor. This is provided in addition to a sensor for sensing a pressure inside the inflatable cuff. The tissue pressure sensor allows for obtaining a quasi-continuous pressure signal which, under certain circumstances, approximates an invasively acquired pulse waveform signal for the patient. This allows various hemodynamic parameters to be derived.

Document US 2015/0320364 A1 describes one example algorithm which enables converting an acquired pressure signal to an approximation of a pulse waveform signal. It is of general advantage to be able to non-invasively measure a patient's pulse waveform, preferably continuously, to avoid using an invasive arterial line. Previous methods in the art have only been able to acquire peripheral (not central) pulse waveform measurements, and there are accuracy issues. For example, traditional known methods include the volume clamp method, which uses a finger cuff, and controls the finger cuff to maintain a constant transmural pressure, so that a constant volume of blood is maintained in the artery.

It was proposed in US 2015/0320364 A1 to use instead measurements obtained using a pressure sensor in a blood pressure measurement cuff.

As a general rule however, it is not possible to simply use the directly measured pressure oscillation signal acquired from the readout of the pressure sensor of the blood pressure cuff. This is because, as a general rule, the relationship between a measured pulse signal (as a function of time) and a true arterial pulse signal (as a function of time) is non-linear with the applied pressure - with the result that the measured pulse signal shape, in general, cannot be expected to match the shape of the arterial pulse wave.

This is thought to be due to the artery wall compliance affecting the linearity of pressure transfer between the arterial pressure and the measured pressure at the sensor.

However, it was realized that, notwithstanding the above general rule, there does exist an applied clamping pressure value where the relationship between the measured pulse signal as a function of time and the true arterial pressure is substantially linear, and thus where the pulse signal shape is more reliably reflective of an arterial pulse waveform shape.

This value was thought to be where the applied clamping pressure equals the mean arterial pressure, because at this clamping pressure the biasing/stress in the arterial wall is minimal, for example substantially 0.

The concept in US 2015/0320364 A1 is to compute a weighted average of a series of pulse wave signals acquired at different respective clamping pressures across a range, and wherein the weightings are related to, or correlated with, the proximity in pressure of a given signal's clamping pressure value to some characteristic clamping pressure which is defined as the clamping pressure for which the internal actual mean arterial pressure equals a certain percentage of the applied clamping pressure.

This characteristic pressure value however cannot be determined, so instead US 2015/0320364 A1 proposes an iterative method for determining the weightings.

Scaling was applied after the general shape of the pulse waveform had been determined using the weighted average signal. The scaling was based on measured values of SBP and DPB, and wherein the pulse waveform shape was scaled in amplitude so that its maximum value matched the SBP and its minimum value matched the DBP.

As a further enhancement of this method, in order to obtain a continuous central blood pressure waveform, rather than just intermittent discrete measures, an additional auxiliary continuous blood pressure measurement device was used (such as the volume clamp finger method), and wherein the continuous waveform output from this auxiliary sensor was adjusted or calibrated according to the intermittent pulse waveform measurements obtained using the more complex method already described above.

However, the algorithm in US 2015/0320364 A1 is very complex.

Thus, a simplified approach to deriving a pulse wave signal and/or for deriving hemodynamic parameters using a continuous pressure signal would be desirable.


SUMMARY OF THE INVENTION

The invention is defined by the claims.

According to examples in accordance with an aspect of the invention, there is provided a method for use in blood pressure measurement by a blood pressure measurement apparatus, wherein the blood pressure measurement apparatus comprises a pressure applicator for use in applying a variable pressure to the tissue of a body part above a blood vessel, and further includes a tissue pressure sensor arranged for sensing a pressure between a surface of the user's body part above the blood vessel and the pressure applicator.

The method comprises: obtaining using the blood pressure measurement apparatus a series of tissue pressure signals (from the tissue pressure sensor), each acquired at a different applied pressure value across a sequence of incrementally increasing or decreasing applied pressure values. The method further comprises deriving from each tissue pressure signal a candidate or sample pulse wave signal based on extracting an AC component of each tissue pressure signal, to thereby derive a series of candidate pulse wave signals, each corresponding to a different applied pressure value across said sequence of applied pressure values. The method further comprises, based on a comparison analysis applied to the waveforms of the series of candidate pulse wave signals, deriving a plot indicative of a change in pulse waveform morphology as a function of applied pressure; identifying an applied pressure value coinciding with each of a set of one or more pre-defined characteristic features of the plot; and deriving one or more arterial blood pressure measures for the subject based on said one or more identified applied pressure values and based on a pre-defined mapping/relationship between the applied pressure values at which said characteristic plot features occur and the one or more arterial blood pressure measures.

Optionally, the method may further comprise generating a data output indicative of the one or more arterial blood pressure measures.

The inventors have recognized that the measured pulse wave signal morphology will change as a function of applied cuff pressure. This is due to the relationship between transmural pressure and arterial wall compliance. Through experimentation and simulation, the inventors have found the surprising result that identifiable characteristic feature points of a plot of the waveform morphology-change as a function of applied pressure are reliably associated with applied pressures which equal certain key blood pressure values, such as systolic blood pressure (SBP), diastolic blood pressure (DBP), and mean arterial pressure (MAP). Thus, the inventors have recognized that this fact can be used to work backwards and, having obtained the plot of the morphology change as a function of applied pressure, work out from this plot the applied pressure values at which the characteristic features occur, and infer that these must correspond to the related blood pressure values.

For example, in some embodiments, the comparison analysis may comprise performing a waveform comparison procedure, comprising performing a comparison between the waveform morphology of each of the plurality of candidate pulse wave signals and at least one other of the plurality of pulse wave signals, so as to measure a change in the morphology of the waveforms as a function of applied pressure.

This provides a novel, and very accurate way of measuring certain hemodynamic parameter measurements, including various measures of blood pressure.

The AC component of each tissue pressure signal provides a pseudo- pulse wave signal (referred to in this document as a candidate or sample pulse wave signal). As explained above, in general the measured pressure signal will only correspond accurately to the waveform shape of the pulse wave signal at a certain range of applied pressures. Thus each measured tissue pressure signal is effectively a candidate for the true pulse wave signal.

In some embodiments, the comparison procedure comprises using one of the measured tissue pressure signals as a reference pulse wave signal and comparing each of the other pulse wave signals against this reference.

For example, in some embodiments, the acquiring of the series of tissue pressure signals comprises: acquiring a reference tissue pressure signal within a defined applied pressure range, and acquiring a series of tissue pressure signals across a sequence of incrementally increasing or decreasing applied pressure values outside of said defined applied pressure range. The deriving of the series of candidate pulse wave signals may comprise: deriving a reference candidate pulse wave signal from the reference tissue pressure signal based on extracting an AC component; and deriving a respective candidate pulse wave signal for each tissue pressure signal acquired outside of said applied pressure range. The method may comprise: for each of the candidate pulse wave signals of the series of signals outside of the pre-defined range, perform a pre-defined comparison procedure comprising a comparison between a waveform morphology of the respective pulse wave signal and the reference pulse wave signal, to derive for the pulse wave signal a respective value of a waveform disparity metric; and plotting the waveform disparity metric as a function of applied tissue pressure to derive said plot indicative of a change in the pulse waveform morphology as a function of applied pressure.

Thus, in this example set of one or more embodiments, one of the obtained pulse wave signals is used as a reference and a waveform disparity metric is derived for each of the other obtained pulse wave signals based on comparison against this reference. This is then plotted as a function of applied pressure.

The particular defined pressure range for which the reference pulse wave signal is acquired might be defined quantitatively in advance, or it might be determined dynamically during the measurement process.

For example, in some embodiments, the pre-defined applied pressure range within which the reference tissue pressure signal is acquired is a range between 0-50 mmHg (= 0 to 6666 Pa), for example between 0-40 mmHg (=0 to 5332 Pa), for example 10-40 mmHg (=1333 to 5332 Pa). Through experimentation and simulation, it has been found by the inventors that a tissue pressure signal acquired at this particular range closely approximates the true invasively acquired pulse pressure waveform for the patient. The value should be greater than zero and less than the defined upper limit for the range.

In some embodiments, the method further comprises generating a data output indicative of the reference pulse wave signal. This provides an output representative of the patient's pulse waveform.

To increase SNR of the obtained reference pulse wave signal, in some embodiments, acquiring the reference tissue pressure signal comprises sampling a tissue pressure signal over a plurality of heart cycles at a constant applied pressure value, and computing an average tissue pressure signal over the multiple heart cycles as the reference tissue pressure signal. Optionally, before averaging, the amplitude and the period of each pulse may be normalized in order to obtain a cleaner averaged pulse.

In some embodiments, instead of a fixed numerical range, the defined applied pressure range is defined according to a characteristic feature or property of the tissue pressure signal which occurs in coincidence with the applied pressure range. The characteristic feature or property may for example be the amplitude of the tissue pressure signal AC component being at or around a maximum. In other words, the reference pulse wave signal is selected as the one of the obtained pulse wave signals which has the highest AC amplitude.

When increasing the applied pressure, a maximum in the amplitude of the AC component is expected to occur in an applied pressure range around the mean arterial pressure (MAP). As will be explained later, from experiment and simulation it has been found that, at an applied pressure approximately equal to mean arterial pressure, the waveform disparity with respect to a true pulse wave for the patient is at or close to zero. The maximum may not necessarily occur exactly at MAP, but in a range close to MAP.

In some examples, the reference candidate pulse wave signal could be selected after the whole measurement sequence has finished, based on identifying the one of the candidate pulse wave signals that possesses the characteristic feature or property.

With regards to performing the comparison process to compute the waveform disparity metric, there are different possible options.

According to at least one set of embodiments, the waveform disparity metric for a given pulse wave signal is computed as an integral of a difference between the respective pulse wave signal and the reference pulse wave signal over a pulse wave period. This example for the waveform disparity metric will be referred to herein as M1.

In this case, the previously mentioned one or more characteristic features of the plot of the waveform disparity metric (M1) may include a zero-crossing point of the plot, and wherein the deriving the one or more blood pressure measures comprises deriving a value for mean arterial blood pressure (MAP) based on the applied pressure value at which said zero-crossing point in the plot occurs.

Additionally or alternatively, in some embodiments, the one or more characteristic features of the plot of the waveform disparity metric (M1) may include: a maximum point of the plot, and wherein the deriving the one or more blood pressure measures comprises deriving a value for diastolic blood pressure (DBP) based on the applied pressure value at which said maximum point in the plot occurs.

Additionally or alternatively, in some embodiments, the one or more characteristic features of the plot of the waveform disparity metric (M1) may include a first inflection point of the plot following the zero crossing point, and wherein the deriving the one or more blood pressure measures comprises deriving a value for systolic blood pressure (SBP) based on the applied pressure value at which said inflection point in the plot occurs.

The reasoning for each of the above proposed characteristic plot features will become clear with reference to simulation data presented in the detailed description later.

According to a further set of embodiments, the waveform disparity metric for a given pulse wave signal may be computed as an integral of a square of the difference between the respective pulse wave signal and the reference pulse wave signal over a pulse wave period. This example for the waveform disparity metric will be referred to herein as M2.

According to this set of embodiments, the one or more characteristic features of the plot of the waveform disparity metric (M2) may include the first local maximum point of the plot, and wherein the deriving the one or more blood pressure measures comprises deriving a value for diastolic blood pressure (DBP) based on the applied pressure value at which said first local maximum point in the plot occurs.

Additionally or alternatively, in some embodiments, the one or more characteristic features of the plot of the waveform disparity metric (M2) includes the first local minimum point following the first local maximum point of the waveform disparity metric plot, and wherein the deriving the one or more blood pressure measurements comprises deriving a value for mean arterial pressure (MAP) based on the applied pressure value at which said minimum point in the plot occurs.

Additionally or alternatively, in some embodiments, the one or more characteristic features of the plot of the waveform disparity metric (M2) include a last inflection point of the plot (or an inflection point following the first local minimum following the local maximum), and wherein the deriving the one or more blood pressure measurements comprises deriving a value for systolic blood pressure (SBP) based on the applied pressure value at which said inflection point in the plot occurs.

The reasoning for each of the above proposed characteristic plot features will become clear with reference to simulation data presented in the detailed description later.

According to a further set of embodiments, the comparison analysis comprises a process of comparing the waveform of each candidate pulse wave signal against that of a preceding candidate pulse wave signal. This approach avoids the need to separately measure a reference pulse wave signal, since each signal is compared against a preceding one.

Thus, in this set of embodiments, the method comprises, for each of the candidate pulse wave signals of the series of candidate pulse wave signals, performing a pre-defined comparison procedure comprising a comparison between a waveform morphology of the respective candidate pulse wave signal and an immediately preceding candidate pulse wave signal in the series, to derive for the pulse wave signal a respective value of a waveform disparity metric (M3); and plotting the waveform disparity metric as a function of applied pressure to derive said plot indicative of a change in the pulse waveform morphology as a function of applied pressure.

According to this set of embodiments, the one or more characteristic features of the plot of the waveform disparity metric (M3) may include a zero-crossing point of the plot, and wherein the deriving the one or more blood pressure measures comprises deriving a value for diastolic blood pressure (DBP) based on the applied pressure value at which said zero-crossing point in the plot occurs.

Additionally or alternatively, in some embodiments, the one or more characteristic features of the plot of the waveform disparity metric (M3) include a first local minimum point of the plot following the zero crossing point, and wherein the deriving the one or more blood pressure measures comprises deriving a value for mean arterial pressure (MAP) based on the applied pressure value at which said local minimum point in the plot occurs.

Additionally or alternatively, in some embodiments, the one or more characteristic features of the plot of the waveform disparity metric (M3) include a first local minimum point of the plot following the zero crossing point, and an inflection point following said local minimum point, and wherein the deriving the one or more blood pressure measures comprises deriving a value for systolic blood pressure (SBP) based on the applied pressure value at which said inflection point in the plot occurs.

The reasoning for each of the above proposed characteristic plot features will become clear with reference to simulation data presented in the detailed description later.

In some embodiments, the method comprises controlling a tissue application pressure cycle of the blood pressure measurement apparatus in which tissue application pressure is incrementally increased or decreased across an application pressure range to realize the sequence of different applied pressures.

In some embodiments, the plot indicative of a change in the pulse waveform morphology as a function of applied pressure is progressively computed in real time as the application pressure cycle progresses, and wherein the application pressure cycle is terminated once all of the set of one or more characteristic plot features have been detected. This has the advantage of minimizing the time required for the measurement process since the process can simply be ended once the needed plot features have all been detected.

The application pressure cycle being terminated means for example that the pressure is relaxed back to zero applied pressure, or minimum applied pressure.

In some embodiments, the blood pressure measurement apparatus includes a cuff for wrapping around a part of the body of a user, and wherein the cuff incorporates the pressure applicator, and wherein the pressure applicator is a pneumatic actuator in the form of an inflatable bladder.

Here, the pressure application cycle comprises an inflation/deflation cycle of the cuff.

In some embodiments, deriving each candidate pulse wave signal further comprises scaling the extracted AC component of the tissue pressure signal, wherein the scaling comprises, for each individual pulse in the signal, maintaining a waveform shape/morphology, but increasing the scale of the waveform such that a maximum point of the waveform matches a pre-measured SBP value, and the minimum point matches a pre-measured DBP value. Thus, scaling is performed. Scaling may additionally be performed in the time dimension such that the period spanned by each individual pulse of the pulse wave signal is scaled to a pre-defined period, for example 1 second. These operations together effectively achieve a normalization of each pulse in the signal in both the amplitude dimension and the time dimension. The phrase 'scaling and normalization' in this document may refer to this pair of operations.

Here, the measured SBP and DBP values may be stored in memory, having been obtained from a previous or the current measurement. They may thus be historical values.

The invention can also be embodied in software form. Thus, another aspect of the invention is a computer program product comprising computer program code configured, when run on a processor operatively coupled to a blood pressure measurement apparatus, to cause the processor to perform a method in accordance with any embodiment recited in this document or any claim of this application.

The invention can also be embodied in hardware.

Thus, another aspect of the invention is a processing device for use in blood pressure measurement by a blood pressure measurement apparatus, wherein the blood pressure measurement apparatus comprises a pressure applicator for use in applying a variable pressure to the tissue of a body part above a blood vessel, and further includes a tissue pressure sensor arranged for sensing a pressure between a surface of the user's body part above the blood vessel and the pressure applicator.

The processing device may comprise: an input/output; and one or more processors, operatively coupled with the input/output and configured to:

> receive at the input/output, from the blood pressure measurement apparatus, a series of tissue pressure signals, each acquired at a different applied pressure value across a sequence of incrementally increasing or decreasing applied pressure values;
> derive from each tissue pressure signal a candidate pulse wave signal based on extracting an AC component of each tissue pressure signal, to thereby derive a series of candidate pulse wave signals, each corresponding to a different applied pressure value across said sequence of applied pressure values;
> based on a comparison analysis applied to the waveforms of the series of candidate pulse wave signals, derive a plot indicative of a change in pulse waveform morphology as a function of applied pressure;
> identify an applied pressure value coinciding with each of a set of one or more pre-defined characteristic features of the plot;
> derive one or more arterial blood pressure measures for the subject based on said one or more identified applied pressure values and based on a pre-defined mapping/relationship between the applied pressure values at which said characteristic plot features occur and the one or more arterial blood pressure measures; and
> optionally generate a data output indicative of the one or more arterial blood pressure measures, and optionally couple the data output to the input/output for export.

Another aspect of the invention is a system, comprising: a processing device as set out above, or in accordance with any embodiment recited in this document or any claim of this application; and a blood pressure measurement apparatus operatively coupled with the processing device, wherein the blood pressure measurement apparatus comprises a pressure applicator for use in applying a variable pressure to the tissue of a body part above a blood vessel, and further includes a tissue pressure sensor arranged for sensing a pressure between a surface of the user's body part above the blood vessel and the pressure applicator.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

> Fig. 1 shows an example blood pressure measurement apparatus;
> Figs. 2-4 illustrate simulated parameters for a simulated blood pressure measurement;
> Fig. 5 illustrates arterial compliance and arterial cross-sectional area as a function of transmural arterial pressure;
> Fig. 6 illustrates a series of comparisons performed between a simulated patient's true pulse waveform and a candidate pulse wave signal obtained from a tissue pressure sensor at a respective applied pressure value;
> Fig. 7 shows a plot of a first example waveform disparity metric, M1, as a function of applied pressure;
> Fig. 8 shows a plot of a second example waveform disparity metric, M2, as a function of applied pressure;
> Fig. 9 shows the plot of Fig. 7 annotated to show three characteristic features of the plot for which the x-values correspond to a set of blood pressure values;

Fig. 10 shows the plot of Fig. 8 annotated to show three characteristic features of the plot for which the x-values correspond to a set of blood pressure values;

Fig. 11 shows a further example plot for metric M1;

Fig. 12 shows a further example plot for metric M2;

Fig. 13 outlines steps of any example method in accordance with one or more embodiments of the invention;

Fig. 14 outlines components of an example processing unit and system according to one or more embodiments of the invention;

Fig. 15 shows a set of signals and parameters acquired from a real patient to illustrate the method proposed in accordance with one or more embodiments of the embodiments;

Fig. 16 illustrates an example plot of applied cuff pressure as a function of time for a first example implementation of the method;

Fig. 17 illustrates an example plot of applied cuff pressure as a function of time for a second example implementation of the method;

Fig. 18 shows a plot of a further example waveform disparity metric, M3, as a function of applied pressure; and

Fig. 19 shows a plot of a further example waveform disparity metric, M4, as a function of applied pressure;

DETAILED DESCRIPTION OF EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for deriving one or more hemodynamic parameters, including one or more measures of blood pressure, based on acquiring a series of sample pulse wave signals at a series of applied pressures using a tissue pressure sensor incorporated between a user's body and a pressure applicator. The hemodynamic parameters are derived based on identifying characteristic points in a derived plot indicative of a change in pulse waveform morphology as a function of applied pressure.

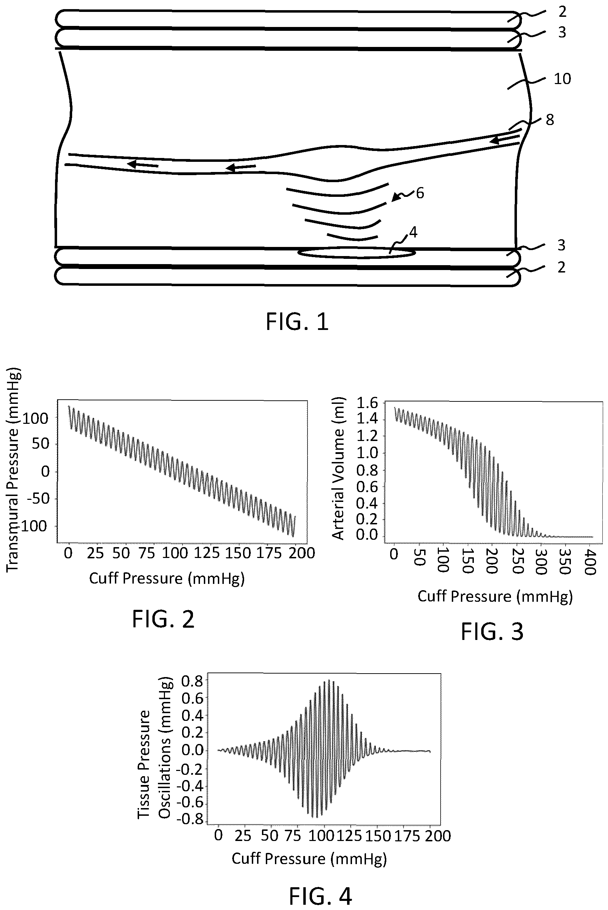

By way of background, Fig. 1 shows a schematic illustration of an example cuff-based hemodynamic parameter measurement apparatus with which embodiments of the present artefact-detection method might advantageously be applied. The apparatus can be used to detect blood pressure and/or other hemodynamic parameter measurements such as cardiac output or stroke volume.

The illustration shows the cuff applied to the upper arm 10 of a patient. An artery 8 of the patient is schematically shown. The apparatus has a design which differs from the most standard cuff-based blood pressure measurement apparatuses in that it includes a dedicated tissue pressure sensor 4 which the cuff acts to hold against the tissue of the skin when the cuff is inflated. The cuff includes a pneumatic actuator in the form of an inflatable bladder 2 for use in changing a pressure applied to the body part 10 by the cuff. The tissue pressure sensor 4 is arranged for sensing a pressure between a surface of the user's body part and the cuff. Independently of the tissue pressure sensor, one or more operational parameters of the pneumatic actuator can be sampled. For example, a pressure inside the inflatable bladder can be sensed. An actuator activity signal indicative of a pumping power level or pumping rate of a pump of the pneumatic actuator can also be sampled.

Standard blood pressure measurement cuffs use the bladder air pressure as the sole measurement signal for sensing blood pressure. By additionally utilizing a tissue pressure sensor 4, the apparatus shown in Fig. 1 can achieve superior quality results and also enables the measurement of advanced hemodynamic parameters such as stroke volume, cardiac output and fluid responsiveness in addition to blood pressure. In particular, in standard air blood pressure cuffs, dampening air is assumed to destroy > 90% of the amplitude and contour of a tissue pressure pulse wave. By contrast, by using a dedicated tissue pressure sensor, the design illustrated in Fig. 1 allows recording high-fidelity (HiFi) artery blood pressure and pulse waveform.

The tissue pressure sensor 4 may be implemented as, e.g. a fluid-filled bag which provides a conforming layer of fluid between the cuff bladder 2 and the surface of the user's body. In this way, the integrated pneumatic actuator enables hydraulic coupling of the tissue pressure sensor 4 to the upper arm tissue. When blood pulses in the artery, this results in a pressure wave 6 which can be detected by the tissue pressure sensor 4. The tissue pressure sensor 4 can be connected to a pressure transducer via a fluid-filled tube/line. The pressure transducer converts the pressure in the fluid into an electrical signal.

The operating principle is based on coupling of the pressure sensor to the body part (e.g. arm) in order to transcutaneously record tissue pressure pulse waves that result from arterial pulsation (e.g. brachial artery). In similar fashion to a conventional upper arm air blood pressure cuff, the cuff compresses the upper arm using an integrated pneumatic actuator with rising clamping pressure. However, the actual compression may be achieved via the narrowing diameter of a rigid circumferential shell.

In the example illustrated in Fig. 1, the cuff includes a shell portion 3, wherein the shell portion is arranged so as to be located between the pneumatic actuator 2 and the body part when the cuff is worn on the body part, and arranged to surround the body part when the cuff is worn. The tissue pressure sensor 4 is arranged so as to be positioned between the shell 3 and the body part when the device is worn on the body part. The shell structure may be relatively stiff. Consequently, the measurement accuracy can be improved, since the tissue pressure measured by tissue pressure sensor 4 is measured against a relatively stiff support, which prevents dampening of the amplitude and shape of the signal. In particular, if a tissue pressure sensor unit 4 is located at least partially between the shell portion 3 and the body part, a high accuracy of the signals measured by the tissue pressure sensor unit can be achieved. With such a configuration of the tissue pressure sensor unit, the shell structure does not absorb or attenuate the arterial pressure signal.

The shell portion 3 may include overlapping sections, and wherein the overlapping sections of the shell portion may move or slide relatively to each other, thereby reducing the diameter of the shell portion responsive to increasing applied pressure by the pneumatic actuator.

If the tissue pressure sensor uses a fluid-based sensing mechanism, as described above, then an initial calibration may be needed in which the pressure transducer, to which the fluid filled pad 4 of the pressure sensor is fluidically coupled, is exposed to atmospheric pressure prior to beginning the measurement. A calibration or correction to account for hydrostatic pressure differences may also be performed.

The design described above permits non-invasive hemodynamic monitoring and enables measurement of blood pressure, as well as cardiac output and other hemodynamic parameters.

For more expansive details about this example hemodynamic parameter measurement apparatus, reference is made to the document EP2953528 A1 which describes the cuff design in more detail.

The blood pressure measurement apparatus utilized as part of, or in combination with, some embodiments of the present invention may include some or all of the components of the hemodynamic parameter measuring system described in the document EP2953528 A1.

Embodiments of the invention might be best understood by first outlining the theoretical background underlying the inventive concepts.

To illustrate this, a simulation was performed by the inventors of an oscillometric measurement. This involved simulating arterial pressure and volume characteristics of a virtual artery for the purposes of the simulation, and these characteristics are shown in Figs. 2-3. Fig. 2 shows the simulated arterial transmural pressure (y-axis; [mmHg]), $P_T$, across the arterial wall as a function of applied pressure to the artery (x-axis; [mmHg]) used for the simulation. Fig. 3 shows the corresponding simulated arterial volume (y-axis; [ml]), $V_p$ , in the virtual artery as a function of applied pressure to the artery (x-axis; [mmHg]). This was simulated using the arterial-transmural pressure - arterial-volume function as follows:

$$V_p(P_T) \; = \; 0.08 \; * \; log(0.03 \; * \; P_T \; + \; 3.3) / \, (1 \; + \; e^{-0.1*P_T}) \; * 10 \qquad (1)$$

In a real measurement, these volume oscillations result in pressure oscillations which are measured by the tissue pressure sensor 4 of the blood pressure measurement apparatus previously referenced, namely a sensor located between the inflatable cuff (or other pressure applicator) and the tissue. For example, it can be implemented by a fluid filled pad at the skin acting as a pressure sensor on top of the brachial artery. This measured tissue pressure signal has an AC component and a DC component. The AC component shows a typical pulse oscillation structure in the frequency band of arterial pulses. The DC component reflects the pressure being applied by the apparatus to the tissue, i.e. the 'applied pressure' as it is referred to in this disclosure. For present purposes, this DC component may be referred to as the tissue pressure ramp or level component, $P_{TPL}$, (also referred to as clamping pressure), and is in the frequency band which is lower than that of typical arterial pulses.

An example simulation of the AC component of the tissue pressure signal corresponding to the volume oscillations of Fig. 3 is shown in Fig. 4. The pressure ramp component ($P_{TPL}$) has been removed to leave just the AC component. In practice, extraction of the AC component of the tissue pressure signal can be done by high-pass filtering. Thus, Fig. 4 shows a simulated recording of tissue pressure signal oscillations (y-axis; [mmHg]) as a function of applied cuff pressure, $P_{ex}$ (x-axis; [mmHg]). The applied cuff pressure increases from 0 mmHg to a value above systolic pressure.

As an approximation, in the forthcoming discussion, the DC component, $P_{TPL}$, of the tissue pressure signal will be considered as equal to the applied pressure (or external pressure) $P_{ex}$ which is applied by the apparatus to the outside of the artery, and thus determining the transmural pressure ($P_T$).

Given that the change in average applied pressure $P_{ex}$ is generally slow ($\sim$ 2mmHg / s) compared to changes in arterial pressures (max $\sim$ 200 mmHg/s), the applied pressure may be assumed to be quasi-static (i.e. constant) over the course of any given pressure pulse.

The volume signal amplitudes are therefore determined in this case by the transmural pressure. As mentioned above, the volume changes are measured by the AC component of the tissue pressure signal.

Mathematically, the obtained pulse signals represented in the tissue pressure signal are dependent upon artery compliance $C(P_T)$, and artery compliance is non-constant as a function of the transmural pressure, $P_T$. Transmural pressure is given by the following:

$$P_T = \mathrm{p_A(t)} - P_{ex} ,$$

where $\mathrm{p_A}(t)$ is the time-dependent arterial blood pressure.

Thus, from the above, it will be recognized that:

- The shape of a pulse wave signal, as measured by the tissue pressure sensor, will vary in dependence upon arterial compliance;
- Arterial compliance varies as a function of transmural pressure; and
- Transmural pressure varies as a function of the applied pressure, $P_{ex}$, applied to the artery.

Thus, it will be recognized that the waveform morphology of a pulse wave signal measured by the tissue pressure sensor will vary as a function of the applied pressure.

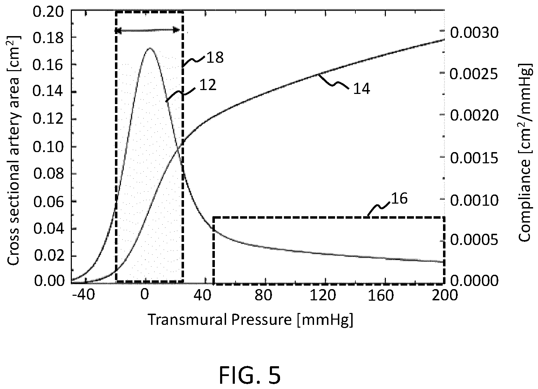

Fig. 5 shows a dependency of arterial compliance, $C(P_T)$ and arterial cross sectional area on varying transmural pressure. The x-axis shows transmural pressure ([mmHg]). The left-hand y-axis shows cross-sectional area ([cm$^2$]); the right-hand y-axis shows arterial compliance ([cm$^2$/mmHg]). Line 12 shows the arterial compliance, $C(P_T)$. Line 14 shows the cross-sectional area.

The compliance $C(P_T)$ is the derivative with respect to $P_T$ of the artery volume:

$$C(P_T) = \frac{dV_p}{dP_T} \text{ with } P_T = p_A(t) - P_{ex}. \tag{2}$$

The volume change is given by:

$$\frac{d}{dt}V_p = C(P_T) * \left(\frac{d}{dt}p_A(t) - \frac{d}{dt}P_{ex}\right) \tag{3}$$

Equation 3 shows the strong dependency of the volume change (as measured in the AC component of the tissue pressure signal) upon the characteristics of arterial compliance, C. Considering the typical measurement conditions, it holds that:

$$\frac{d}{dt}P_{ex} \ll \frac{d}{dt}p_A(t). \tag{4}$$

The impact of the artery compliance on the measured tissue pressure signal is dominated by the behavior of $C(P_T)$.

The application of the above considerations to embodiments of the present invention will now be discussed.

From the above considerations, the inventors have recognized at least two main implications.

A first main implication is that, by looking to the simulations, it is possible to identify specific ranges of transmural pressure, and thus specific ranges of applied pressure, $P_{ex}$, where the impact of arterial compliance on the morphology of a measured pulse wave signal (measured using the tissue pressure sensor) is minimal. It has thus been recognized by the inventors that by measuring the tissue pressure signal AC component within these minimal impact regions, one can obtain a pulse wave signal that most closely matches the true morphology of the patient actual pulse waveform.

A second main implication, which follows from the first, is that, by plotting the change in pulse wave morphology as a function of applied pressure, $P_{ex}$, one can identify those applied pressure values at which characteristic features in the plot occur, and infer from this the value of key blood pressure measures.

Looking to Fig. 5, reference is made to the area indicated with box 16. This is a region of the compliance, C, curve 12 at high transmural pressure values. High transmural pressure corresponds to low average applied pressure $P_{ex}$. In this region, the impact of compliance is minimal because compliance is approximately constant as a function of transmural pressure. Thus, for this region of high transmural pressure and low applied pressure, the measured tissue pressure signal over a pulse period closely corresponds to the true pulse waveform signal of the individual:

$$\frac{d}{dt}V_p = C * \frac{d}{dt}p_A(t) \quad with \ C \sim constant \tag{5}$$

Therefore, in this region of low applied pressure, the measured tissue pressure signal is proportional to the arterial pressure signal in shape.

Thus, the measured tissue pressure signal in this region of low applied pressure closely matches the pulse waveform of the patient. As will be explained below, the inventors have furthermore identified a quantitative range of applied pressure values corresponding to region 16 of Fig. 5, meaning that if the tissue pressure signal is sampled in this region, the resulting waveform can be taken as an accurate estimation of the patient's pulse waveform. Thus, this goes to the first implication mentioned above.

In contrast, for pressure levels around zero transmural pressure, the compliance function, $C(P_T)$ shows a large peak with large dependency upon transmural pressure. This region of large impact of transmural pressure is indicated by box 18. In this region, the measured tissue pressure waveform morphology is strongly impacted by the transmural pressure:

$$\frac{d}{dt}V_p = C(P_T) * \frac{d}{dt}p_A(t) \quad with \ largely \ varying \ C \tag{6}$$

Furthermore, the compliance dependency function peaks around a transmural pressure of zero. A transmural pressure of zero means that the applied pressure, $P_{ex}$, approximately matches the (mean) arterial pressure. It follows from this that the region of applied pressures at which the measured tissue pressure waveform morphology can be expected to exhibit peak morphology variation is around a region where the applied pressure is close to at least one measure of arterial pressure. From this, it will be recognized that by plotting morphology change of measured tissue pressure signals as a function of applied pressure and looking for certain key characteristic points in the plot of morphology change (e.g. maxima, minima, inflections or zero crossings), the values of certain key blood pressure measures can be identified. This therefore goes to the second main implication mention above. This has been investigated in more detail by the inventors as will now be explained further.

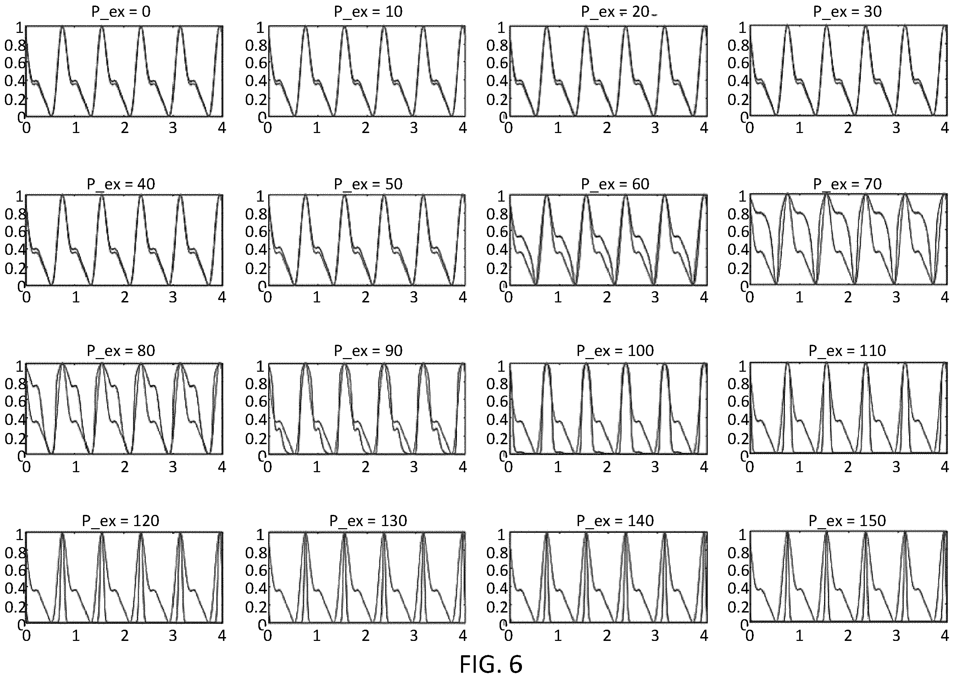

The impact of arterial blood pressure on the morphology of tissue pressure signals has been simulated and an illustrative sample of the results are visualized in Fig. 6. Each of the individual graphs of Fig. 6 shows a comparison of a respective simulated pulse waveform for simulated arterial pressure having a systolic pressure value (SBP) of 123 mmHg and a diastolic pressure value (DBP) of 90 mmHg. Thus, all of the graphs correspond to the same assumed arterial blood pressure wave. However, for each graph, a different applied pressure value ($P_{ex}$) was simulated and a corresponding tissue pressure signal simulated. The result is that each graph shows a comparison between (a) a simulated AC component of a measured tissue pressure signal over a single pulse period for one of a range of applied pressure, P_ex, values (ranging from 0-150 mmHg) and (b) a simulated true pulse waveform corresponding to arterial pressure varying between SBP = 123 mmHg and DBP = 90 mmHg. The x-axis shows time in seconds. The comparison is illustrated simply by overlaying waveform (a) atop waveform (b). This is presented in Fig. 6 for illustrative purposes only.

To enable the comparison of waveforms, the simulated signals have been normalized to the range [0 1] and scaled according to the SBP and DBP values mentioned above. The pulse period has been assumed to be constant.

To compute a quantification of each comparison, different options are possible.

By way of one example, a comparison of the scaled and normalized pulse waveforms (per pulse k) can be achieved by computing a waveform disparity metric (M 1) as an integral of a difference between the signals over a pulse wave period, $T_k = T_{k\_end} - T_{k\_start}$, where $T_k$ is the pulse wave period for pulse $k$, $T_{k\_start}$ is the time of the start of the pulse $k$ and $T_{k\_end}$ is the time at the end of the pulse $k$. By way of a further example, a comparison of the scaled and normalized pulse waveforms (per pulse k) can be achieved by computing a waveform disparity metric (M2) equal to an integral of a square of the difference between the waveforms, again over a pulse wave period, $T_k = T_{k\_end} - T_{k\_start}$.

These can be quantified as follows:

$$M1 = \frac{1}{K}\sum_k \frac{1}{T_{k\_end}-T_{k\_start}} \int_{T_{k\_start}}^{T_{k\_end}} \left(s_1(t) - s_2\left(t - T_{k\_start}\right)\right) dt \qquad (7a)$$

or

$$M1 = \frac{1}{\sum_k(T_{k\_end}-T_{k\_start})}\sum_k \int_{T_{k\_start}}^{T_{k\_end}} \left(s_1(t) - s_2\left(t - T_{k\_start}\right)\right) dt \qquad (7b)$$

and

$$M2 = \frac{1}{K}\sum_k \frac{1}{T_{k\_end}-T_{k\_start}} \int_{T_{k\_start}}^{T_{k\_end}} \left(s_1(t) - s_2\left(t - T_{k\_start}\right)\right)^2 dt \qquad (8a)$$

or

$$M2 = \frac{1}{\sum_k(T_{k\_end}-T_{k\_start})}\sum_k \int_{T_{k\_start}}^{T_{k\_end}} \left(s_1(t) - s_2\left(t - T_{k\_start}\right)\right)^2 dt \qquad (8b)$$

For the performed simulation, for each comparison, $s_1$ corresponds to the simulated tissue pressure signal at one of the applied pressures, and $s_2$ corresponds to the simulated true pulse waveform.

In the above expressions, $K$ is the total number of pulses used to determine the disparity metric. For consecutive pulses it typically holds that $T_{k+1\_start} = T_{k\_end}$, i.e., the end of the preceding pulse is the start of the next pulse. This holds in case of no missing pulses due to e.g. misdetections. The simulated true pulse wave signal $s_2(t)$ is considered to run from 0 to $T_k = T_{k\_end} - T_{k\_start}$. In a simplest case, each disparity metrics M1 and M2 may be computed for a single individual pulse only, and thus the summations in Equations (7a), (7b) and (8a), (8b) would contain only a single element. In this case, Equations (7a) and (b) would reduce to the same expression and Equations (8a) and (8b) would reduce to the same expression. Furthermore, in case the duration of each pulse has been scaled to a same duration period, e.g. 1 s, the difference $T_{k\_end} - T_{k\_start}$ is equal to that scaled duration period, e.g. 1 s, for each individual pulse $k$.

It is noted that, as a variation on the above, an M1 metric could be determined from the absolute difference between $s_1(t)$ and $s_2(t)$, i.e., by integrating $|s_1(t) - s_2(t - T_{k\_start})|$ instead of

$$\left(s_1(t) - s_2\left(t - T_{k\_start}\right)\right).$$

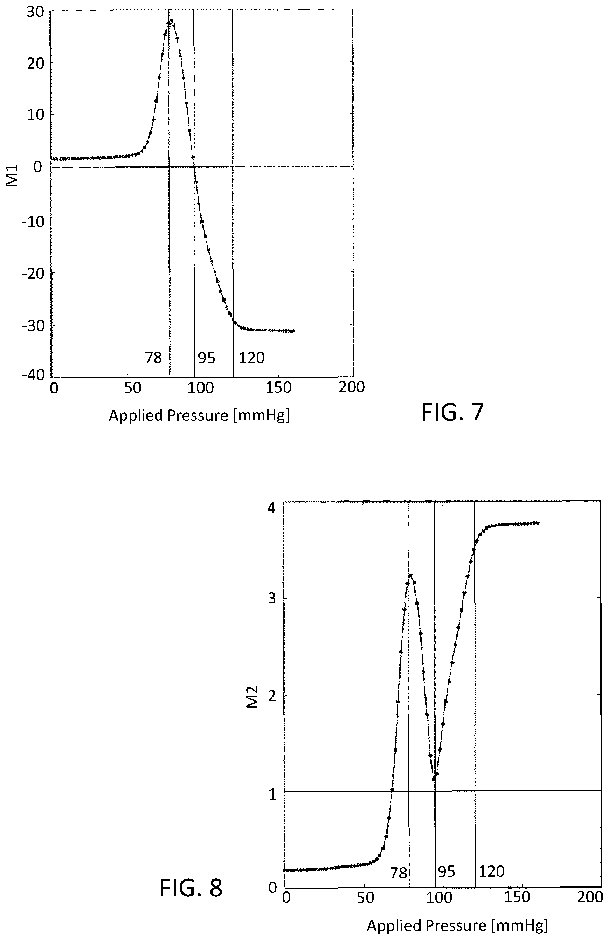

Fig. 7 shows an example plot of the first waveform disparity metric M1 (y-axis) as a function of applied pressure (x-axis; [mmHg]), where the summations in M1 contain only a single element: i.e, K=1 and the disparity is determined at each applied pressure value for a single individual pulse only. In this case, the two expressions given for M1 above reduce to the same single expression

$$\frac{1}{T_{k\_end}-T_{k\_start}} \int_{T_{k\_start}}^{T_{k\_end}} \left(s_1(t) - s_2\left(t - T_{k\_start}\right)\right) dt.$$

Fig. 8 shows a plot of the second waveform disparity M2 (y-axis) as a function of applied pressure (x-axis; [mmHg]), where the summations in M2 contain only a single element: i.e., K=1 and the disparity is determined at each applied pressure value for a single individual pulse only. In this case, the two expressions given for M2 above reduce to the same single expression

$$\frac{1}{T_{k\_end}-T_{k\_start}} \int_{T_{k\_start}}^{T_{k\_end}} \left(s_1(t) - s_2\left(t - T_{k\_start}\right)\right)^2 dt$$

For Fig 7, differences have directly been determined between the pulses. For Fig. 8, differences have been determined between pulses which had been normalized and scaled for more exact comparison. Hence the difference in range of the vertical axes of Fig. 7 and Fig. 8.

Indicated on each graph are indicative vertical lines which show the locations along each plot where the applied pressure (x-axis) was equal to the stimulated diastolic blood pressure (DBP = 78 mmHg), mean arterial blood pressure (MAP = 95 mmHg) and systolic blood pressure (SBP = 120 mmHg) of the simulated artery.

It can be seen that on each graph, the points where the applied cuff pressure (x-axis) matched these three respective values of blood pressure correspond to characteristic features of the respective plots for M1 (Fig. 7) and M2 (Fig. 8).

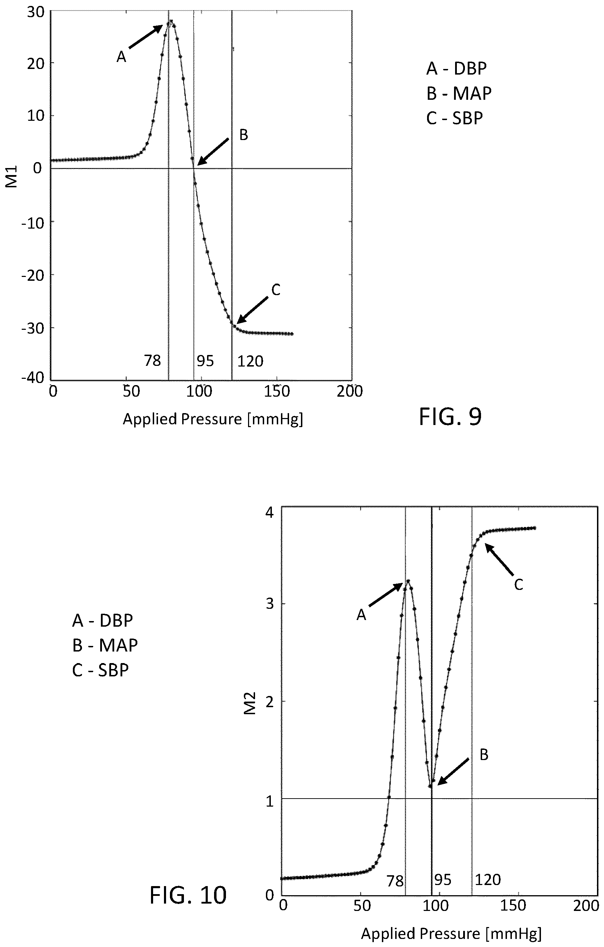

This is illustrated more explicitly in Fig. 9 and Fig. 10 which respectively show a first (A), second (B) and third (C) feature of each plot, where the first feature in each plot coincides with an applied pressure value which equals the diastolic blood pressure (DBP), the second feature (B) of each plot coincides with an applied pressure value which equals the mean arterial blood pressure (MAP) and the third feature (C) of each plot coincides with an applied pressure value which is equal to the systolic blood pressure (SBP). The features follow one another in sequence.

In the plot for M1: the plot feature (A) coinciding with applied pressure equal to DBP is a first local maximum point of the plot; the plot feature (B) coinciding with applied pressure equal to MAP is a zero-crossing point of the plot; and the plot feature (C) coinciding with applied pressure equal to SBP is a first inflection point of the plot following the zero crossing point.

In the plot for M2: the plot feature (A) coinciding with applied pressure equal to DBP is a first local maximum point of the plot; the plot feature (B) coinciding with applied pressure equal to MAP is a first local minimum point of the plot; and the plot feature (C) coinciding with applied pressure equal to SBP is a final inflection point of the plot, or a next inflection point following said first local minimum point (B).

A further observation apparent from each plot is that, for applied pressure values below about 40-50 mmHg, each of the disparity metrics M1, M2 have only a very small, roughly constant value. From this it can be concluded that a sample pulse wave signal measured with the tissue pressure sensor within this applied pressure range can be expected to closely match the waveform morphology of the true pulse wave signal for the patient (in view of the fact that the plots of M1 and M2 in Fig. 9 and Fig. 10 represent disparity in the waveform morphology of a respective pulse waveform measured with the tissue pressure sensor at a given applied pressure with the simulated true pulse waveform for the patient).

From the above, it has been recognized by the inventors that these technical observations could be applied in reverse in order to:

(a) obtain a pulse waveform for a patient which closely matches the true pulse waveform morphology (i.e. an "A-line like" waveform), based on acquiring a sample pulse waveform with the tissue pressure sensor within an applied pressure range for which the waveform disparity metric is known to be low (e.g. in the range 0-40 mmHg or 0-50 mmHg, or around the point B corresponding to mean arterial pressure).
(b) derive unknown values of DBP, MAP and SBP based on generating a plot of a waveform disparity metric such as M1 or M2 from an acquired sequence of sample pulse wave signals measured with a tissue pressure sensor at a sequence of different applied pressure values, each compared against a reference pulse wave signal acquired at an applied pressure value where waveform disparity is known to be low (e.g. in the range 0-40 mmHg or 0-50 mmHg or around the point B corresponding to mean arterial pressure).

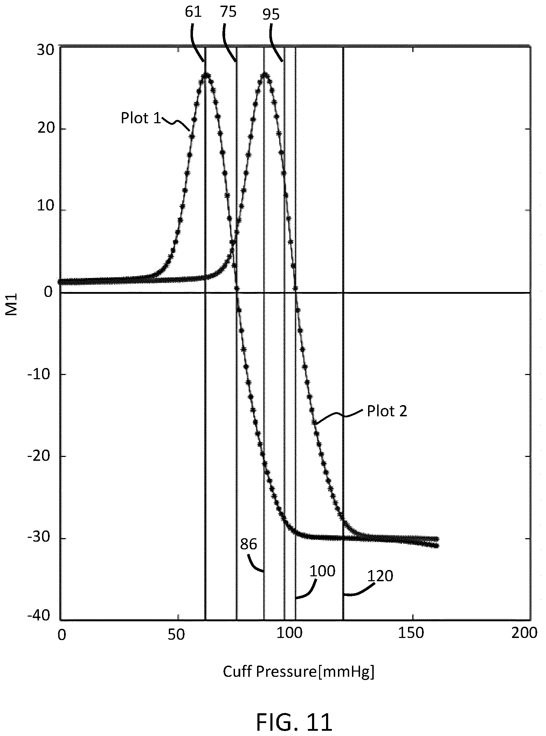

This was tested and confirmed with a further plot for M1, labelled Plot 1 in Fig. 11. Plot 1 was generated for simulated blood pressure values: DBP=61 mmHg; MBP=75 mmHg, SBP=95 mmHg. Plot 2 in Fig. 11 shows, for comparison, a plot generated for simulated blood pressure values: DBP=86 mmHg, MBP=100 mmHg, SBP=120 mmHg. For the lower blood pressure of Plot 1, the curve shifts to the left. It can be seen that, for Plot 1 and Plot 2, the applied pressure values coincide with the simulated DBP, MAP and SBP values at the same characteristic plot features A, B and C labelled in Fig. 9.

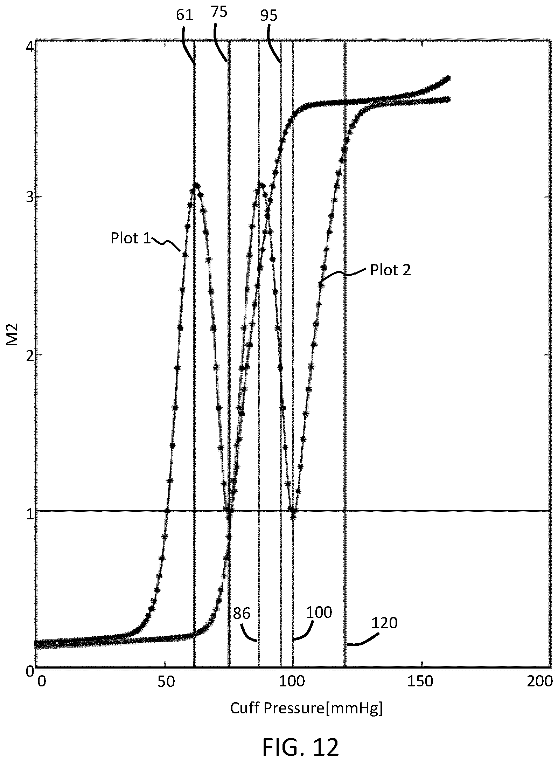

Fig. 12 shows a further plot for M2, labelled Plot 1 in Fig. 12. Plot 1 was generated for simulated blood pressure values: DBP=61 mmHg; MBP=75 mmHg, SBP=95 mmHg Plot 2 in Fig. 12 shows, for comparison, a plot generated for simulated blood pressure values: DBP=86 mmHg, MBP=100 mmHg, SBP=120 mmHg. For the lower blood pressure of Plot 1, the curve shifts to the left. It can be seen that, for Plot 1 and Plot 2, the applied pressure values coincide with the simulated DBP, MAP and SBP values at the same characteristic plot features A, B and C labelled in Fig. 10.

It is the proposal of the inventors to apply these considerations in practice to enable inference of blood pressure values by measuring sample pulse wave signals with the tissue pressure sensor as a function of varying applied pressure and plotting waveform morphology variation as a function of applied pressure. Using the above theoretical findings, characteristic points in these plots can then be used to infer blood pressure values.

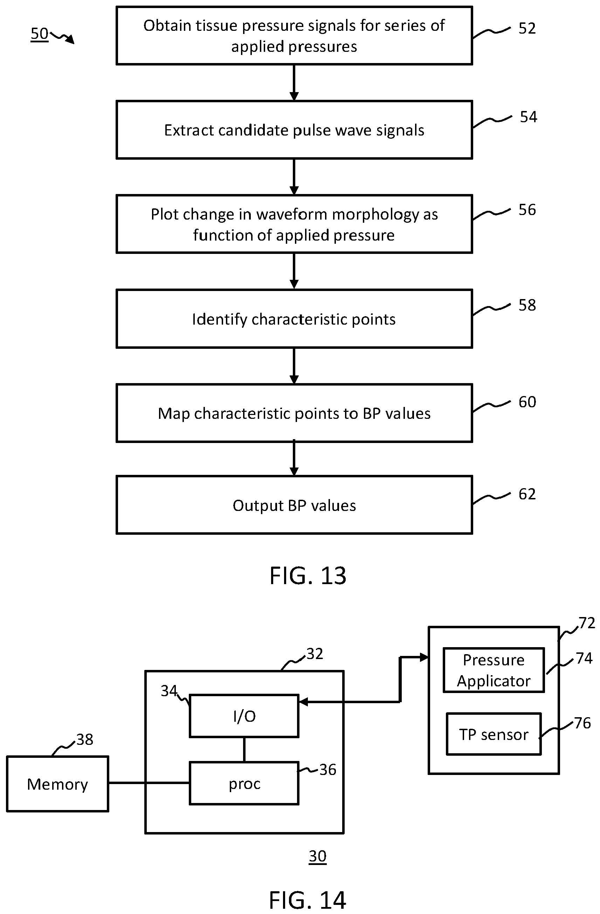

Fig. 13 outlines in block diagram form steps of an example computer-implemented method 50 according to one or more embodiments. The steps will be recited in summary, before being explained further in the form of example embodiments.

Provided is a method 50 for use in blood pressure measurement by a blood pressure measurement apparatus, wherein the blood pressure measurement apparatus comprises a pressure applicator for use in applying a variable pressure to the tissue of a body part above a blood vessel, and further includes a tissue pressure sensor arranged for sensing a pressure between a surface of the user's body part above the blood vessel and the pressure applicator.

The method comprises obtaining 52, using the blood pressure measurement apparatus, a series of tissue pressure signals, each acquired at a different applied pressure value across a sequence of incrementally increasing or decreasing applied pressure values.

The method further comprises deriving 54 from each tissue pressure signal a candidate (or sample or pseudo-) pulse wave signal based on extracting an AC component of each tissue pressure signal, to thereby derive a series of candidate pulse wave signals, each corresponding to a different applied pressure value across said sequence of applied pressure values.

The method further comprises, based on a comparison analysis applied to the waveforms of the series of candidate pulse wave signals, deriving 56 a plot indicative of a change in pulse waveform morphology as a function of applied pressure.

The method further comprises identifying 58 an applied pressure value coinciding with each of a set of one or more pre-defined characteristic features of the plot.

The method further comprises deriving 60 one or more arterial blood pressure measures for the subject based on said one or more identified applied pressure values and based on a pre-defined mapping or relationship between the applied pressure values at which said characteristic plot features occur and the one or more arterial blood pressure measures.

The method preferably may further comprise generating 62 a data output indicative of the one or more arterial blood pressure measures.

As noted above, the method can also be embodied in hardware form, for example in the form of a processing unit which is configured to carry out a method in accordance with any example or embodiment described in this document, or in accordance with any claim of this application.

To further aid understanding, Fig. 14 presents a schematic representation of an example processing unit 32 configured to execute a method in accordance with one or more embodiments of the invention. The processing unit is shown in the context of a system 30 which comprises the processing unit. The processing unit alone represents an aspect of the invention. The system 30 is another aspect of the invention. The provided system does not have to comprise all of the illustrated hardware components; it may just comprise a subset of them.

The processing unit 32 comprises one or more processors 36 configured to perform a method in accordance with that outlined above, or in accordance with any embodiment described in this document or any claim of this application. In the illustrated example, the processing unit further comprises an input/output 34.

In the illustrated example of Fig. 14, the system 30 further comprises a blood pressure measurement apparatus 72. The blood pressure measurement apparatus 72 may comprise a pressure applicator 74 for use in applying a variable pressure to the tissue of a body part above a blood vessel, and further includes a tissue pressure sensor 76 arranged for sensing a pressure between a surface of the user's body part above the blood vessel and the pressure applicator. The blood pressure measurement apparatus may comprise a cuff for being worn by the patient, and wherein the pressure applicator 74 takes the form of a pneumatic actuator comprising an inflatable bladder, and wherein a varying inflation level of the bladder varies the applied pressure.

In some embodiments, the system 30 further comprises a user interface (not shown) for displaying the derived blood pressure values and/or other values or signals.

The input/output 34 may be adapted for receiving the previously mentioned series of tissue pressure signals, and optionally for outwardly communicating the data output indicative of the one or more arterial blood pressure measures. The tissue pressure signals might alternatively be received from a datastore, or from an intermediary communication device, such as a hub server or a network node.

For example, the input/output 34 may be adapted for wired or wireless connection to one or more external units. These might include the blood pressure measurement apparatus.

The system 30 may further comprise a memory 38 for storing computer program code (i.e. computer-executable code) which is configured for causing the one or more processors 36 of the processing unit 32 to perform the method as outlined above, or in accordance with any embodiment described in this disclosure, or in accordance with any claim.

The blood pressure measurement apparatus may optionally take the form of the blood pressure measurement device detailed in EP2953528 A1 and described earlier in this document.

As mentioned previously, the invention can also be embodied in software form. Thus another aspect of the invention is a computer program product comprising code means configured, when run on a processor, to cause the processor to perform a method in accordance with any example or embodiment of the invention described in this document, or in accordance with any claim of this patent application.

Embodiments of this invention are based on the observations derived from the simulations outlined in detail above. To test these principles in practice, the proposed method was run on real data obtained from real patients.

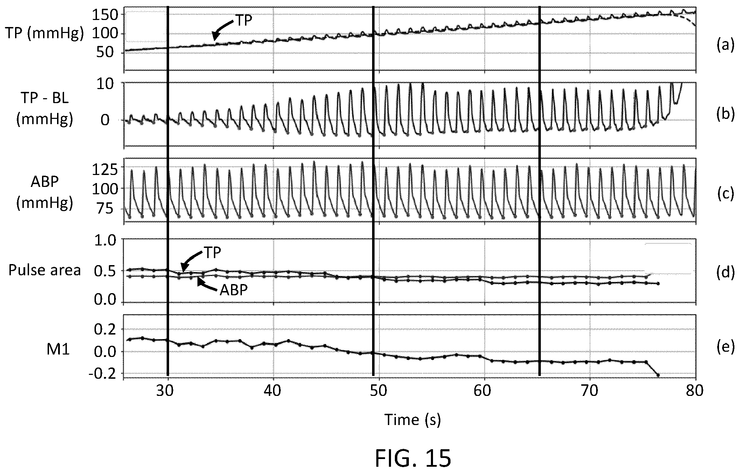

By way of illustration, Fig. 15 shows measurement data for an example patient. Each chart shows a trace for the relevant quantity (y-axis) as a function of time (x-axis; seconds).

Chart (a) shows a signal output from a tissue pressure (TP) sensor (y-axis; mmHg) as a function of time as an applied pressure is incrementally increased. The baseline of the chart (a) represents the value of the applied pressure. Chart (b) shows the extracted AC component of the tissue pressure signal of chart (a), i.e. the tissue pressure signal of chart (a) with the baseline (BL) removed. This therefore provides a candidate pulse wave signal spanning a sequence of individual pulses for the patient. For purposes of comparison, chart (c) shows the true arterial blood pressure for the patient measured simultaneously with the tissue pressure signal, and derived from an invasive measurement means. This signal is not acquired during actual performance of the method according to the invention, and was acquired for purposes of proof of concept. Chart (d) corresponds to an area under each pulse of a normalized version of chart (b) and chart (c) - i.e. shows a plot of the area under each pulse of the signal (b) after normalization (labelled TP) and a plot of the area under each arterial blood pressure pulse after normalization (labelled ABP). Chart (e) represents a computed value for the previously discussed waveform disparity metric M1 for each of the sequence of candidate pulse wave signals represented by the pulses of chart (b) (after normalization):

$$M1\big(T_{k\_end}\big) = \frac{1}{T_{k\_end}-T_{k\_start}} \int_{T_{k\_start}}^{T_{k\_end}} \big(s_1(t) - s_2(t)\big)dt$$

, i.e., Equation (7a) or (7b), computed in each case with only a single pulse in the summation, where $s_1$ corresponds to the relevant normalized candidate pulse wave signal pulse in chart (b) and $s_2$ corresponds to the corresponding normalized candidate pulse wave signal pulse in chart (c). That is, chart (e) shows the differences between the pulse areas shown in chart (d).

It may be observed that, in Fig. 15, the disparity metric M1 shows a zero-crossing at a time where the applied pressure (which is represented by the baseline of chart (a)) is at a value approximately equal to the MAP value (i.e. around 100 mmHg). The first local maximum in M1 occurs around a time where the applied pressure is at a value approximately equal to the DBP (i.e. around 70 mmHg) and an inflection point in M1 occurs around a time where the applied pressure value is approximately equal to the SBP (i.e. around 125 mmHg). It is noted that the first local maximum and the inflection point are slightly difficult to observe in the particular graphical representation of Fig. 15, due to the particular scaling which has been used. For ease of reading, vertical lines have been placed along the charts at the three characteristic points along the plot of M1 where the applied pressure (baseline of chart (a)) corresponds to the blood pressure measures DBP, MAP and SBP respectively, from left to right.

Applying the above-described technical insights, as a more general principle, and with reference again to the method outlined in summary form in Fig. 13, according to one group of embodiments, it is proposed to perform the method in the following way.

The acquiring of the series of tissue pressure signals may comprise: acquiring a reference tissue pressure signal within a defined applied pressure range, and acquiring a series of tissue pressure signals across a sequence of incrementally increasing or decreasing applied pressure values outside of said applied pressure range. The defined applied pressure range may be a pre-defined numerical range (e.g. the range 0-40 mmHg or 0-50 mmHg), or may be determined dynamically based on identifying a characteristic feature or property of the tissue pressure signal which occurs in coincidence with the applied pressure range (e.g. maximum amplitude, which typically occurs around MAP). It may be a range known or estimated to coincide with a measured candidate pulse wave signal which closely matches the morphology of the true pulse wave signal. The simulations discussed above have shown that one suitable range is for instance 0-50 mmHg or 0-40 mmHg where waveform disparity (M1, M2) was found to be consistently at its lowest. Another option is to define the defined applied pressure range according to a characteristic feature or property of the tissue pressure signal which occurs in coincidence with the applied pressure range, for example the characteristic feature or property might be the amplitude of the tissue pressure signal AC component being at a maximum. As shown above with reference to Fig. 9 and Fig. 10, at an applied pressure approximately equal to mean arterial pressure, the waveform disparity was found to be minimal (zero in Fig. 9, and at a local minimum in Fig. 10).

Deriving the series of candidate pulse wave signals may comprise: deriving a candidate pulse wave signal from the tissue pressure signal based on extracting an AC component, and deriving a respective candidate pulse wave signal for each tissue pressure signal acquired outside of said applied pressure range.

The method may comprise: for each of the candidate pulse wave signals of the series of signals outside of the pre-defined range, performing a pre-defined comparison procedure comprising a comparison between a waveform morphology of the respective pulse wave signal and the reference pulse wave signal, to derive for the pulse wave signal a respective value of a waveform disparity metric (e.g. M1, M2); and plotting the waveform disparity metric as a function of applied tissue pressure to derive said plot indicative of a change in the pulse waveform morphology as a function of applied pressure.

The defined pressure range within which the reference tissue pressure signal is acquired may be a range between 0-50 mmHg, for example between 0-40 mmHg (=0 to 5332 Pa), for example 10-40 mmHg (=1333 to 5332 Pa).

The method may further comprises generating a data output indicative of the reference pulse wave signal. Thus might be used as an estimated measure of the patient's true pulse wave ('A-line' line pulse wave signal).

In some embodiments, the deriving of each candidate pulse wave signal may further comprise scaling the extracted AC component for each candidate pulse wave signal, wherein the scaling comprises maintaining a waveform shape/morphology, but increasing the scale of the waveform such that a maximum point of the waveform matches a measured SBP value, and the minimum point matches a measured DBP value. The measured SBP and DBP values might be historical values measured for the patient for example.

In some embodiments, accuracy can be improved by generating the reference tissue pressure signal based on an average over multiple tissue pressure signal pulses all acquired at a same applied pressure value. In other words, it is generated by sampling the tissue pressure signal over a plurality of heart cycles at a constant applied pressure value, and computing an average tissue pressure signal over the multiple heart cycles as the reference tissue pressure signal. This increases signal-to-noise ratio. Optionally, before averaging, the amplitude and the period of the pulse could be normalized in order to obtain a cleaner averaged pulse.

According to one set of embodiments, the waveform disparity metric is the metric M1 discussed above.

Thus here, the waveform disparity metric (M1) for a given pulse wave signal is computed as an integral (per pulse, $k$) of a difference between the respective pulse wave signal and the reference pulse wave signal over a pulse wave period for each pulse $k$, $T_k = T_{k\_end} - T_{k\_start}$. This can be stated mathematically as:

$$M1 = \frac{1}{K} \sum_k \frac{1}{T_{k\_end} - T_{k\_start}} \int_{T_{k\_start}}^{T_{k\_end}} \left( s_1(t) - s_2\left(t - T_{k\_start}\right) \right) dt$$

or

$$M1 = \frac{1}{\sum_k (T_{k\_end} - T_{k\_start})} \sum_k \int_{T_{k\_start}}^{T_{k\_end}} \left( s_1(t) - s_2\left(t - T_{k\_start}\right) \right) dt$$

where, $s_1$ corresponds to a given candidate pulse wave signal at one of the applied pressures, and $s_2$ corresponds to the reference pulse wave signal, and $K$ is the total number of pulses used to determine the disparity metric. For consecutive pulses it typically holds that $T_{k+1\_start} = T_{k\_end}$, i.e. the end of the preceding pulse is the start of the next pulse. This holds in case of no missing pulses due to e.g. misdetections. The reference pulse wave signal $s_2(t)$ is considered to run from 0 to $T_k = T_{k\_end} - T_{k\_start}$. In a simplest case, the disparity metric M1 may be determined per individual pulse, so that the summations in the equations above contain only a single element (K=1). In this case, the two possible equations defined above for M1 reduce to the same. Furthermore, in case the duration of each pulse has been scaled to a same duration period, e.g. 1 s, the difference $T_{k\_end} - T_{k\_start}$ is equal to that scaled duration period, e.g. 1 s, for each individual pulse $k$.

It is noted that, as a variation on the above, an M1 metric could be determined from the absolute difference between $s_1(t)$ and $s_2(t)$, i.e., by integrating $|s_1(t) - s_2(t - T_{k\_start})|$ instead of

$$\left( s_1(t) - s_2\left(t - T_{k\_start}\right) \right).$$

Where the waveform disparity metric is M1, the one or more characteristic features of the plot of the waveform disparity metric may include a zero-crossing point of the plot, and wherein the deriving the one or more blood pressure measures comprises deriving a value for mean arterial blood pressure (MAP) based on the applied pressure value at which said zero-crossing point in the plot occurs. For an explanation of this, see Fig. 7 and Fig. 9 above and the associated discussion.

Where the waveform disparity metric is M1, one or more characteristic features of the plot of the waveform disparity metric may include a maximum point of the plot (e.g. a first local maximum of the plot), and wherein the deriving the one or more blood pressure measures comprises deriving a value for diastolic blood pressure (DBP) based on the applied pressure value at which said maximum point in the plot occurs. For an explanation of this, see Fig. 7 and Fig. 9 above and the associated discussion.

Where the waveform disparity metric is M1, the one or more characteristic features of the plot of the waveform disparity metric may include a first inflection point of the plot following the zero crossing point, and wherein the deriving the one or more blood pressure measures comprises deriving a value for systolic blood pressure (SBP) based on the applied pressure value at which said inflection point in the plot occurs. For an explanation of this, see Fig. 7 and Fig. 9 above and the associated discussion.

According to at least one set of embodiments, additionally or alternatively, the waveform disparity metric may be the metric M2 discussed above.

Thus here, the waveform disparity metric M2 for a given pulse wave signal is computed as an integral (per pulse $k$) of a square of the difference between the respective pulse wave signal and the reference pulse wave signal over a pulse wave period for each pulse $k$, $T_k = T_{k\_end} - T_{k\_start}$.

This may be defined mathematically as follows:

$$M2 = \frac{1}{K}\sum_k \frac{1}{T_{k\_end} - T_{k\_start}} \int_{T_{k\_start}}^{T_{k\_end}} \left(s_1(t) - s_2\bigl(t - T_{k\_start}\bigr)\right)^2 dt$$

or

$$M2 = \frac{1}{\sum_k (T_{k\_end} - T_{k\_start})} \sum_k \int_{T_{k\_start}}^{T_{k\_end}} \left(s_1(t) - s_2\bigl(t - T_{k\_start}\bigr)\right)^2 dt$$

where, $s_1$ corresponds to a given candidate pulse wave signal at one of the applied pressures, and $s_2$ corresponds to the reference pulse wave signal, and $K$ is the total number of pulses used to determine the disparity metric. For consecutive pulses it typically holds that $T_{k+1\_start} = T_{k\_end}$, i.e., the end of the preceding pulse is the start of the next pulse. This holds in case of no missing pulses due to e.g. misdetections. The reference pulse wave signal $s_2(t)$ is considered to run from 0 to $T_k = T_{k\_end} - T_{k\_start}$. In a simplest case, the disparity metric M2 may be determined per individual pulse, so that the summations in the equations above contain only a single element (K=1). In this case, the two possible equations defined above for M2 reduce to the same. Furthermore, in case the duration of each pulse has been scaled to a same duration period, e.g. 1 s, the difference $T_{k\_end} - T_{k\_start}$ is equal to that scaled duration period, e.g. 1 s, for each individual pulse $k$.

Where the waveform disparity metric is M2, the one or more characteristic features of the plot of the waveform disparity metric may include the first local maximum point of the plot, and wherein the deriving the one or more blood pressure measures comprises deriving a value for diastolic blood pressure (DBP) based on the applied pressure value at which said first local maximum point in the plot occurs. For an explanation of this, see Fig. 8 and Fig. 10 above and the associated discussion.

Where the waveform disparity metric is M2, the one or more characteristic features of the plot of the waveform disparity metric may include the first local minimum point following the first local maximum point of the waveform disparity metric plot, and wherein the deriving the one or more blood pressure measurements comprises deriving a value for mean arterial pressure (MAP) based on the applied pressure value at which said minimum point in the plot occurs. For an explanation of this, see Fig. 8 and Fig. 10 above and the associated discussion.

Where the waveform disparity metric is M2, the one or more characteristic features of the plot of the waveform disparity metric may include an inflection point following the first local minimum (or a last inflection point of the plot), and wherein the deriving the one or more blood pressure measurements comprises deriving a value for systolic blood pressure (SBP) based on the applied pressure value at which said inflection point in the plot occurs. For an explanation of this, see Fig. 8 and Fig. 10 above and the associated discussion.

With regards to the acquisition of the reference candidate pulse wave signal, this can be performed at different times relative to the sequence of incrementally increasing or decreasing applied pressure values. One option is to acquire the reference candidate pulse wave signal as a separate step at a start of the measurement process before the sequence of incrementally increasing or decreasing applied pressure values begins. The relevant applied pressure could be applied to the body part and the reference pulse wave signal measured.

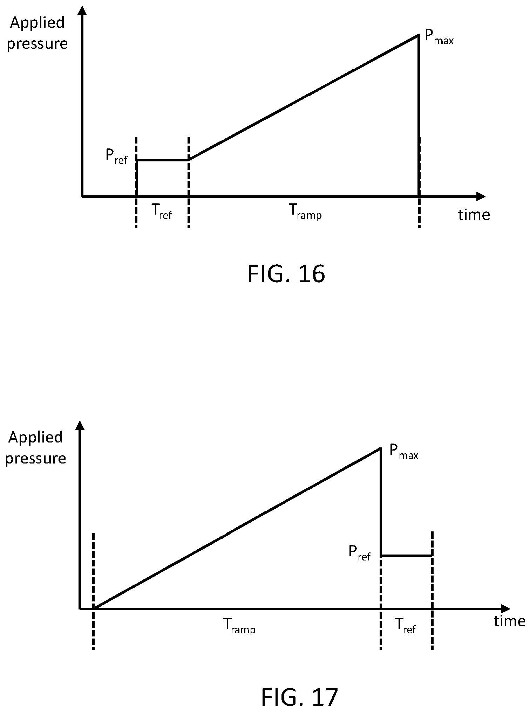

This is illustrated schematically in Fig. 16. As illustrated, the performed method can be understood as comprising two phases: a first phase in which the reference candidate pulse wave signal is measured at the reference applied pressure, $P_{ref}$, and a second phase in which the applied pressure is ramped up from $P_{ref}$ to an upper pressure value $P_{max}$. During the second phase, a respective candidate pulse wave signal is measured at a series of applied pressure values along the upward ramp, as has been described in detail above. The time period spanned by the first phase is labelled as $T_{ref}$ and the time period spanned by the second phase is labelled as $T_{ramp}$. As discussed above, the reference candidate pulse wave signal may be measured over a plurality of pulse cycles, and an average taken of the plurality of pulse cycles, to thereby increase SNR.

Another option is to acquire the reference candidate pulse wave signal as part of the sequence of incrementally increasing or decreasing applied pressure values; in other words it is acquired as simply one of the series of candidate pulse wave signals.

A further option is to acquire the reference pulse wave signal at an end of the measurement process, after the sequence of applied pressure values has finished. In the latter case, the comparison procedure in which the values of the waveform disparity metric are computed would need to be performed 'off-line' after the measurement process has concluded.

This is illustrated schematically in Fig. 17. Again, this comprises a first phase and a second phase. However, in this example, the first phase comprises ramping up the applied pressure from a minimum value (e.g. zero) to an upper pressure value, $P_{max}$. A respective candidate pulse wave signal is measured at each of a series of applied pressure values along the ramp, as already discussed. During the second phase, the reference candidate pulse wave signal is measured at the reference applied pressure, $P_{ref}$, The time period spanned by the first phase is labelled as $T_{ramp}$ and the time period spanned by the second phase is labelled as $T_{ref}$.

Instead of metric M1 and M2, an alternative waveform disparity metric may be used which is based on comparing each acquired candidate pulse wave signal with a preceding candidate pulse wave signal. This avoids any need to acquire a reference pulse wave signal, as is required for computing M1 or M2.

Thus, according to one or more embodiments, the method 50 may comprise: for each of the candidate pulse wave signals of the series of candidate pulse wave signals, performing a pre-defined comparison procedure comprising a comparison between a waveform morphology of the respective candidate pulse wave signal and an immediately preceding candidate pulse wave signal in the series, to derive for the pulse wave signal a respective value of a waveform disparity metric (M3, M4); and plotting the waveform disparity metric as a function of applied pressure to derive said plot indicative of a change in the pulse waveform morphology as a function of applied pressure.

In accordance with this approach one example waveform disparity metric M3 is as follows. M3 can be understood as the waveform disparity of each candidate pulse wave signal as compared with a previous candidate pulse wave signal. It can be computed as the integral of the differences of pulse wave signal k compared to pulse wave signal k-1. In effect, it corresponds to the derivative of M1 with respect to applied pressure.

Thus here, the waveform disparity metric (M3) for a given pulse wave signal is computed as an integral of a difference between the respective pulse wave signal and the immediately preceding pulse wave signal over a pulse wave period, $T_k = T_{k\_end} - T_{k\_start}$.

This can be stated mathematically as:

$$M3 = \frac{1}{K}\sum_k \frac{1}{T_{k\_end}-T_{k\_start}} \int_{T_{k\_start}}^{T_{k\_end}} \left(s_1(t) - s_2\left(t - T_{k\_start}\right)\right) dt$$

or

$$M3 = \frac{1}{\sum_k (T_{k\_end}-T_{k\_start})} \sum_k \int_{T_{k\_start}}^{T_{k\_end}} \left(s_1(t) - s_2\left(t - T_{k\_start}\right)\right) dt$$

where $s_1$ corresponds to a given candidate pulse wave signal at one of the applied pressures, and $s_2$ corresponds to the preceding pulse wave signal at a preceding applied pressure, and K is the total number of pulses used to determine the disparity metric. For consecutive pulses it typically holds that $T_{k+1\_start} = T_{k\_end}$, i.e., the end of the preceding pulse is the start of the next pulse. This holds in case of no missing pulses due to e.g. misdetections. The reference pulse wave signal $s_2(t)$ is considered to run from 0 to $T_k = T_{k\_end} - T_{k\_start}$. In a simplest case, the disparity metric M3 may be determined per individual pulse, so that the summations in the equations above contain only a single element (K=1). In this case, the two possible equations defined above for M3 reduce to the same. Furthermore, in case the duration of each pulse has been scaled to a same duration period, e.g. 1 s, the difference $T_{k\_end}-T_{k\_start}$ is equal to that scaled duration period, e.g. 1 s, for each individual pulse $k$.

The pulse wave signal and the immediately preceding pulse wave signal may be normalized in amplitude and scaled in time for an improved comparison and to assure that both pulse wave signals last equally long in time.

Additionally or alternatively, a waveform disparity metric M4 could be used, where M4 is computed as the integral of the squared differences of pulse wave signal k compared to pulse wave signal k-1. This is effectively a first derivative of M2 with respect to applied pressure.

Thus here, the waveform disparity metric M4 for a given pulse wave signal is computed as an integral of a square of the difference between the respective pulse wave signal and the immediately preceding pulse wave signal over a pulse wave period, $T_k = T_{k\_end} - T_{k\_start}$.

This may be defined mathematically as follows:

$$M4 = \frac{1}{K}\sum_k \frac{1}{T_{k\_end} - T_{k\_start}} \int_{T_{k\_start}}^{T_{k\_end}} \left(s_1(t) - s_2\left(t - T_{k\_start}\right)\right)^2 dt$$

or

$$M4 = \frac{1}{\sum_k (T_{k\_end} - T_{k\_start})} \sum_k \int_{T_{k\_start}}^{T_{k\_end}} \left(s_1(t) - s_2\left(t - T_{k\_start}\right)\right)^2 dt$$

where $s_1$ corresponds to a given candidate pulse wave signal at one of the applied pressures, and $s_2$ corresponds to the preceding pulse wave signal at a preceding applied pressure, and $K$ is the total number of pulses used to determine the disparity metric. For consecutive pulses it typically holds that $T_{k+1\_start} = T_{k\_end}$, i.e., the end of the preceding pulse is the start of the next pulse. This holds in case of no missing pulses due to e.g. misdetections. The reference pulse wave signal $s_2(t)$ is considered to run from 0 to $T_k = T_{k\_end} - T_{k\_start}$. In a simplest case, the disparity metric M4 may be determined per individual pulse, so that the summations in the equations above contain only a single element (K=1). In this case, the two possible equations defined above for M4 reduce to the same. Furthermore, in case the duration of each pulse has been scaled to a same duration period, e.g. 1 s, the difference $T_{k\_end} - T_{k\_start}$ is equal to that scaled duration period, e.g. 1 s, for each individual pulse $k$.

The pulse wave signal and the immediately preceding pulse wave signal can be normalized in amplitude and scaled in time for an improved comparison and to assure that both pulse wave signals last equally long in time.

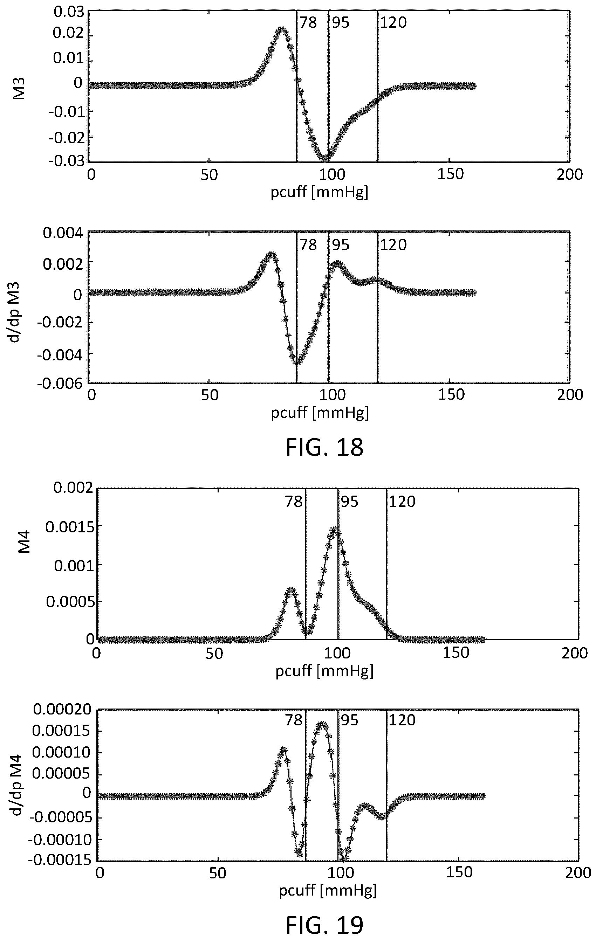

Fig. 18 (top) shows an example plot of the waveform disparity metric M3 (y-axis) as a function of applied pressure (x-axis; [mmHg]).

Fig. 19 (top) shows a plot of the second waveform disparity M4 (y-axis) as a function of applied pressure (x-axis; [mmHg]).

Each plot was generated based on a simulation using simulated blood pressure values: DBP=78 mmHg, MAP=95 mmHg, SBP=120 mmHg .

Indicated on each graph are indicative vertical lines which show the locations along each plot where the applied pressure (x-axis) was equal to the simulated diastolic blood pressure (DBP = 78 mmHg), mean arterial blood pressure (MAP = 95 mmHg) and systolic blood pressure (SBP = 120 mmHg) of the simulated artery.

It can be seen that, on each graph, the points where the applied cuff pressure (x-axis) matched these three respective values of blood pressure correspond to certain characteristic features of the respective plots for M3 and M4.

In particular, for M3 (Fig. 18 - top) it can be seen that a zero-crossing point of the plot coincides with the applied pressure value (x-axis) which is equal to the DBP (=78 mmHg); a first local minimum point of the plot following the zero crossing point coincides with the applied pressure value which is equal to the MAP (=95 mmHg); and an inflection point following said local minimum point coincides with the applied pressure value which is equal to the SBP (=120 mmHg).

With regards to M4 (Fig. 19 - top), it can be seen that a first local minimum point of the plot coincides with an applied pressure value (x-axis) which is equal to the DBP (=78 mmHg); a first local maximum point of the plot following the first local minimum coincides with an applied pressure value which is equal to the MAP (=95 mmHg); and an inflection point following said local maximum point coincides with an applied pressure value equal to the systolic blood pressure (SBP). The inflection point marks a point in the signal at which the gradient changes from negative to substantially flat.

Following from the above, with regards to M3, the characteristic plot features may be more formally stated as follows.

The one or more characteristic features of the plot of the waveform disparity metric (M3) may include a zero-crossing point of the plot, and wherein the deriving the one or more blood pressure measures comprises deriving a value for diastolic blood pressure (DBP) based on the applied pressure value at which said zero-crossing point in the plot occurs.

The one or more characteristic features of the plot of the waveform disparity metric (M3) may include a first local minimum point of the plot following the zero crossing point, and wherein the deriving the one or more blood pressure measures comprises deriving a value for mean arterial pressure (MAP) based on the applied pressure value at which said local minimum point in the plot occurs.

The one or more characteristic features of the plot of the waveform disparity metric (M3) may include a first local minimum point of the plot following the zero crossing point, and an inflection point following said local minimum point, and wherein the deriving the one or more blood pressure measures comprises deriving a value for systolic blood pressure (SBP) based on the applied pressure value at which said inflection point in the plot occurs.

With regards to M4, the characteristic plot features may be as follows.

The one or more characteristic features of the plot of the waveform disparity metric (M4) may include a first local minimum point of the plot, and wherein the deriving the one or more blood pressure measures comprises deriving a value for diastolic blood pressure (DBP) based on the applied pressure value at which said first local minimum point in the plot occurs.

The one or more characteristic features of the plot of the waveform disparity metric (M4) may include a first local maximum point of the plot following the said first local minimum point, and wherein the deriving the one or more blood pressure measures comprises deriving a value for mean arterial pressure (MAP) based on the applied pressure value at which said first local maximum point in the plot occurs.

The one or more characteristic features of the plot of the waveform disparity metric (M4) may include a first local maximum point of the plot following the first local minimum point, and an inflection point following said local maximum point, and wherein the deriving the one or more blood pressure measures comprises deriving a value for systolic blood pressure (SBP) based on the applied pressure value at which said inflection point in the plot occurs. The inflection point marks a point in the signal at which the gradient changes from negative to substantially flat.

Each of Fig. 18 (bottom) and Fig. 19 (bottom) also show a first derivative with respect to cuff pressure of the respective metric (M3 and M4), which helps illustrate the characteristic points in the plots of M3 and M4 in terms of the gradient. In some examples, the set of characteristic points might be identified for example based on first taking a first derivative (d/dp) of the respective metric (M3 or M4) and then finding characteristic points of this derivative plot. For example, maxima and minima of M3 or M4 both show as zero-crossing points in the respective derivative signal, while the zero-crossing point in M3 shows as a minimum in the derivative d(M3)/dp.

The accuracy of measures M3 and M4 can be improved by scaling pulses in time as well as in amplitude, so they all have a pulse period of e.g. 1 second and an amplitude of e.g. 1.

Note that, in accordance with any of the above outlined methods (M1, M2, M3, M4) interpolation can be applied to the plot of the respective waveform disparity metric to enable more precise identification of the relevant characteristic plot features (e.g. maxima/minima, zero crossings, inflection points). In other words, if the relevant plot feature occurs between two points of the plot, interpolation can be applied to identify a more precise value of the applied pressure coinciding with the characteristic plot feature. Interpolation can be performed for example using cubic spline interpolation methods or any other interpolation method.

In accordance with some embodiments, the method may be extended to permit real-time tracking of blood pressure. This can be achieved by first executing the method 50 as already discussed above, to derive initial values for DBP, MAP and SBP and also to derive a measure of the reference pulse wave signal. In addition, the plot of M1 or M2 as a function of applied pressure can be derived and stored. At this point, the method may comprise adjusting the applied cuff pressure to a fixed (steady) value, $P_{fixed}$, equal to one of the measured initial values for DBP, MAP and SBP. The tissue pressure sensor can be used to continuously or recurrently measure a candidate pulse wave signal, $s_1(t)$. This measured signal, $s_1(t)$, can then be used to compute a value for one of the waveform disparity metrics M1 or M2 discussed above, using the already acquired reference pulse wave signal, $s_2(t)$. The obtained value for M1 or M2 can be used to find the corresponding plot point along the already derived and stored plot for M1 or M2 vs applied pressure.

The value for M1 or M2 can be repeatedly or continuously re-computed using new measured $s_1$ waveforms, and any changes in M1 or M2 monitored. Responsive to any detected change in M1 or M2, the cuff applied pressure is adjusted so as to keep M1 or M2 at the original characteristic plot point along the originally derived and stored plot for M1 or M2 vs applied pressure. Thus, a feedback loop is implemented whereby applied pressure is automatically adjusted to maintain the value of M1 or M2 at its original characteristic plot point along the originally derived and stored plot for M1 or M2 vs applied pressure. The applied pressure value at any given time may then be used as a real-time estimate of the blood pressure value (corresponding to the characteristic plot point). By way of example, if the applied cuff pressure is originally set to a fixed (steady) value, $P_{fixed}$, equal to the MAP, and the metric M1 is being used, then the applied pressure is simply adjusted continuously or recurrently to keep M1 = 0, and the real-time applied pressure can be read as a real-time value for MAP.

In other words, based on the measured M1 or M2 plot, first a fixed (steady) applied cuff pressure value, $P_{fixed}$, is selected corresponding to the desired characteristic features of the M1 or M2 plot corresponding to DBP, MAP or SBP. Subsequently, M1 or M2 values are continuously or recurrently computed at the fixed (steady) applied cuff pressure value, $P_{fixed}$. If any changes in the new M1 or M2 values are found, the fixed (steady) applied cuff pressure value, $P_{fixed}$, is adjusted in order to keep the values for M1 or M2 constant at one of the desired characteristic features of the M1 or M2 plot. That is, the value of M1 or M2 at cuff pressure $P_{fixed}$ is monitored and the applied cuff pressure $P_{fixed}$ is continuously adjusted to keep the value of M1 or M2 constant at the relevant characteristic point along the plot of M1 or M2. This allows the applied cuff pressure $P_{fixed}$ to be used to track one of the blood pressure values of DBP, MAP or SBP.

The tracking of any of the DBP, MAP or SBP using this approach might in some embodiments be performed for a limited amount of time, after which a new full inflation/deflation cycle is executed in order to obtain a new M1 or M2 plot. The said limited amount of time may be a predefined time period, e.g., 10 minutes, 20 minutes or 30 minutes. Alternatively, re-determination of the full M1 or M2 plot might be triggered automatically, responsive to a change in DBP, MAP or SBP from the initial values of DBP, MAP or SBP which exceeds a pre-defined threshold.

In accordance with one or more embodiments, the method comprises controlling a tissue application pressure cycle of the blood pressure measurement apparatus 72 in which tissue application pressure is incrementally increased or decreased across an application pressure range to realize the sequence of different applied pressures.

Advantageously, in accordance with some embodiments, the plot indicative of a change in the pulse waveform morphology as a function of applied pressure can be progressively computed in real time as the application pressure cycle progresses, and wherein the application pressure cycle is terminated once all of the set of one or more characteristic plot features have been detected.

The application pressure cycle being terminated means for example that the pressure is relaxed back to zero applied pressure, or minimum applied pressure. This has the benefit that the time period for which pressure is applied to the patient's body part (e.g. arm) can be minimized.

For example, with reference to Fig. 9, in a case where the method involves plotting waveform disparity metric M1, once plot features A, B and C have all been detected, the applied pressure might be reduced back to zero to end the measurement cycle.

The blood pressure measurement apparatus may in some embodiments comprise a cuff for wrapping around a part of the body of a user, and wherein the cuff incorporates the pressure applicator, and wherein the pressure applicator is a pneumatic actuator in the form of an inflatable bladder. Here, the pressure application cycle comprises an inflation/deflation cycle of the cuff.

Embodiments of the invention described above employ a processing device. The processing device may in general comprise a single processor or a plurality of processors. It may be located in a single containing device, structure or unit, or it may be distributed between a plurality of different devices, structures or units. Reference therefore to the processing device being adapted or configured to perform a particular step or task may correspond to that step or task being performed by any one or more of a plurality of processing components, either alone or in combination. The skilled person will understand how such a distributed processing device can be implemented. The processing device includes a communication module or input/output for receiving data and outputting data to further components.

The one or more processors of the processing device can be implemented in numerous ways, with software and/or hardware, to perform the various functions required. A processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

**Claims**

1. A method for use in blood pressure measurement by a blood pressure measurement apparatus, wherein the blood pressure measurement apparatus comprises a pressure applicator for use in applying a variable pressure to the tissue of a body part above a blood vessel, and further includes a tissue pressure sensor arranged for sensing a pressure between a surface of the user's body part above the blood vessel and the pressure applicator:
the method comprising:

   obtaining using the blood pressure measurement apparatus a series of tissue pressure signals, each acquired at a different applied pressure value across a sequence of incrementally increasing or decreasing applied pressure values;

deriving from each tissue pressure signal a candidate pulse wave signal based on extracting an AC component of each tissue pressure signal, to thereby derive a series of candidate pulse wave signals, each corresponding to a different applied pressure value across said sequence of applied pressure values;

based on a comparison analysis applied to the waveforms of the series of candidate pulse wave signals, deriving a plot indicative of a change in pulse waveform morphology as a function of applied pressure;

identifying an applied pressure value coinciding with each of a set of one or more pre-defined characteristic features of the plot; and

deriving one or more arterial blood pressure measures for the subject based on said one or more identified applied pressure values and based on a pre-defined mapping/relationship between the applied pressure values at which said characteristic plot features occur and the one or more arterial blood pressure measures;

generating a data output indicative of the one or more arterial blood pressure measures.

2. The method of claim 1,

wherein the acquiring of the series of tissue pressure signals comprises:

acquiring a reference tissue pressure signal within a defined applied pressure range, and

acquiring a series of tissue pressure signals across a sequence of incrementally increasing or decreasing applied pressure values outside of said applied pressure range; and

deriving the series of candidate pulse wave signals comprises:

deriving a reference candidate pulse wave signal from the reference tissue pressure signal based on extracting an AC component; and

deriving a respective candidate pulse wave signal for each tissue pressure signal acquired outside of said applied pressure range;

wherein the method comprises:

for each of the candidate pulse wave signals of the series of signals outside of the pre-defined range, perform a pre-defined comparison procedure comprising a comparison between a waveform morphology of the respective pulse wave signal and the reference pulse wave signal, to derive for the pulse wave signal a respective value of a waveform disparity metric (M1, M2); and

plotting the waveform disparity metric as a function of applied tissue pressure to derive said plot indicative of a change in the pulse waveform morphology as a function of applied pressure.

3. The method of claim 2, wherein the method further comprises generating a data output indicative of the reference pulse wave signal.

4. The method of claim 2 or 3, where the defined applied pressure range within which the reference tissue pressure signal is acquired is a range between 0-50 mmHg (= 0 to 6666 Pa), for example 0-40 mmHg (=0 to 5332 Pa), for example 10-40 mmHg (=1333 to 5332 Pa).

5. The method of claim 2 or 3, wherein the defined applied pressure range is defined according to a characteristic feature or property of the tissue pressure signal which occurs in coincidence with the applied pressure range, wherein the characteristic feature or property is the amplitude of the tissue pressure signal AC component being at a maximum.

6. The method of any of claims 2-5, wherein the waveform disparity metric (M1) for a given pulse wave signal is computed based on an integral of a difference between the respective pulse wave signal and the reference pulse wave signal over a pulse wave period.

7. The method of claim 6,

wherein the one or more characteristic features of the plot of the waveform disparity metric (M1) include a zero-crossing point of the plot, and wherein the deriving the one or more blood pressure measures comprises deriving a value for mean arterial blood pressure (MAP) based on the applied pressure value at which said zero-crossing point in the plot occurs; and/or

wherein the one or more characteristic features of the plot of the waveform disparity metric (M1) include a maximum point of the plot, and wherein the deriving the one or more blood pressure measures comprises deriving a value for diastolic blood pressure (DBP) based on the applied pressure value at which said maximum point in the plot occurs; and/or

wherein the one or more characteristic features of the plot of the waveform disparity metric (M1) include a first inflection point of the plot following the zero crossing point, and wherein the deriving the one or more blood pressure measures comprises deriving a value for systolic blood pressure (SBP) based on the applied pressure value at which said inflection point in the plot occurs.

8. The method of any of claims 2-5, wherein the waveform disparity metric (M2) for a given pulse wave signal is computed based on an integral of a square of the difference between the respective pulse wave signal and the reference pulse wave signal over a pulse wave period.

9. The method of claim 8,

wherein the one or more characteristic features of the plot of the waveform disparity metric (M2) includes the first local maximum point of the plot, and wherein the deriving the one or more blood pressure measures comprises deriving a value for diastolic blood pressure (DBP) based on the applied pressure value at which said first local maximum point in the plot occurs; and/or

wherein the one or more characteristic features of the plot of the waveform disparity metric (M2) includes the first local minimum point following the first local maximum point of the waveform disparity metric plot, and wherein the deriving the one or more blood pressure measurements comprises deriving a value for mean arterial pressure (MAP) based on the applied pressure value at which said minimum point in the plot occurs; and/or

wherein the one or more characteristic features of the plot of the waveform disparity metric (M2) include a last inflection point of the plot, and wherein the deriving the one or more blood pressure measurements comprises deriving a value for systolic blood pressure (SBP) based on the applied pressure value at which said inflection point in the plot occurs.

10. The method of claim 1,
wherein the method comprises:

for each of the candidate pulse wave signals of the series of candidate pulse wave signals, performing a predefined comparison procedure comprising a comparison between a waveform morphology of the respective candidate pulse wave signal and an immediately preceding candidate pulse wave signal in the series, to derive for the pulse wave signal a respective value of a waveform disparity metric (M3); and

plotting the waveform disparity metric as a function of applied pressure to derive said plot indicative of a change in the pulse waveform morphology as a function of applied pressure.

11. The method of claim 10,

wherein the one or more characteristic features of the plot of the waveform disparity metric (M3) include a zero-crossing point of the plot, and wherein the deriving the one or more blood pressure measures comprises deriving a value for diastolic blood pressure (DBP) based on the applied pressure value at which said zero-crossing point in the plot occurs; and/or

wherein the one or more characteristic features of the plot of the waveform disparity metric (M3) include a first local minimum point of the plot following the zero crossing point, and wherein the deriving the one or more blood pressure measures comprises deriving a value for mean arterial pressure (MAP) based on the applied pressure value at which said local minimum point in the plot occurs; and/or

wherein the one or more characteristic features of the plot of the waveform disparity metric (M3) include a first local minimum point of the plot following the zero crossing point, and an inflection point following said local minimum point, and wherein the deriving the one or more blood pressure measures comprises deriving a value for systolic blood pressure (SBP) based on the applied pressure value at which said inflection point in the plot occurs.

12. The method of any of claims 1-11, wherein

the method comprises controlling a tissue application pressure cycle of the blood pressure measurement apparatus in which tissue application pressure is incrementally increased or decreased across an application pressure range to realize the sequence of different applied pressures; and

wherein the plot indicative of a change in the pulse waveform morphology as a function of applied pressure is progressively computed in real time as the application pressure cycle progresses, and wherein the application pressure cycle is terminated once all of the set of one or more characteristic plot features have been detected.

13. The method of any preceding claim, wherein the blood pressure measurement apparatus includes a cuff for wrapping around a part of the body of a user, and wherein the cuff incorporates the pressure applicator, and wherein the pressure applicator is a pneumatic actuator in the form of an inflatable bladder.

14. A computer program product comprising computer program code configured, when run on a processor operatively coupled to a blood pressure measurement apparatus, to cause the processor to perform a method in accordance with any of claims 1-13.

15. A processing device for use in blood pressure measurement by a blood pressure measurement apparatus, wherein the blood pressure measurement apparatus comprises a pressure applicator for use in applying a variable pressure to the tissue of a body part above a blood vessel, and further includes a tissue pressure sensor arranged for sensing a pressure between a surface of the user's body part above the blood vessel and the pressure applicator, the processing device comprising:

an input/output; and
one or more processors, operatively coupled with the input/output and configured to:

receive at the input/output, from the blood pressure measurement apparatus, a series of tissue pressure signals, each acquired at a different applied pressure value across a sequence of incrementally increasing or decreasing applied pressure values;

derive from each tissue pressure signal a candidate pulse wave signal based on extracting an AC component of each tissue pressure signal, to thereby derive a series of candidate pulse wave signals, each corresponding to a different applied pressure value across said sequence of applied pressure values;

based on a comparison analysis applied to the waveforms of the series of candidate pulse wave signals, derive a plot indicative of a change in pulse waveform morphology as a function of applied pressure;

identify an applied pressure value coinciding with each of a set of one or more pre-defined characteristic features of the plot;

derive one or more arterial blood pressure measures for the subject based on said one or more identified applied pressure values and based on a pre-defined mapping/relationship between the applied pressure values at which said characteristic plot features occur and the one or more arterial blood pressure measures; and

generate a data output indicative of the one or more arterial blood pressure measures.

16. A system, comprising:

a processing device as claimed in claim 15; and
a blood pressure measurement apparatus operatively coupled with the processing device, wherein the blood pressure measurement apparatus comprises a pressure applicator for use in applying a variable pressure to the tissue of a body part above a blood vessel, and further includes a tissue pressure sensor arranged for sensing a pressure between a surface of the user's body part above the blood vessel and the pressure applicator.

EP 4 400 039 A1

FIG. 1

FIG. 2

FIG. 3

FIG. 4

FIG. 5

FIG. 6

FIG. 7

FIG. 8

A - DBP
B - MAP
C - SBP

**FIG. 9**

A - DBP
B - MAP
C - SBP

**FIG. 10**

FIG. 11

FIG. 12

50 ⟶

```
┌─────────────────────────────────────────────┐
│  Obtain tissue pressure signals for series of │ ～ 52
│              applied pressures                │
└─────────────────────────────────────────────┘
                       │
                       ▼
┌─────────────────────────────────────────────┐
│       Extract candidate pulse wave signals    │ ～ 54
└─────────────────────────────────────────────┘
                       │
                       ▼
┌─────────────────────────────────────────────┐
│     Plot change in waveform morphology as     │ ～ 56
│          function of applied pressure         │
└─────────────────────────────────────────────┘
                       │
                       ▼
┌─────────────────────────────────────────────┐
│          Identify characteristic points       │ ～ 58
└─────────────────────────────────────────────┘
                       │
                       ▼
┌─────────────────────────────────────────────┐
│       Map characteristic points to BP values  │ ～ 60
└─────────────────────────────────────────────┘
                       │
                       ▼
┌─────────────────────────────────────────────┐
│               Output BP values                │ ～ 62
└─────────────────────────────────────────────┘
```

FIG. 13

```
                              ┌──────────────────────┐
                          ～32 │              ～72     │
   ┌──────────────────────────┤   ┌──────────────┐～74 │
   │      34～  ┌──────────┐   │   │   Pressure   │    │
   │           │   I/O    │◄──┼───┤  Applicator  │    │
   │           └──────────┘   │   └──────────────┘    │
┌──────────┐ 38│      │       │   ┌──────────────┐～76 │
│  Memory  │───┤  ┌──────────┐│   │   TP sensor  │    │
│          │   │  │   proc   │～36│  └──────────────┘    │
└──────────┘   │  └──────────┘│   └──────────────────────┘
   38～         └──────────────┘
                      30
```

FIG. 14

FIG. 15

FIG. 16

FIG. 17

FIG. 18

FIG. 19

Europäisches
Patentamt

European
Patent Office

Office européen
des brevets

# EUROPEAN SEARCH REPORT

Application Number

EP 23 15 1041

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/298188 A1 (DANA ALEXANDRA [IL] ET AL) 3 October 2019 (2019-10-03) * paragraphs [0047], [0059] – [0097]; claims; figures * ----- | 1-16 | INV. A61B5/022 A61B5/00 |
| X | US 2017/360313 A1 (BAEK DAVID BOETTCHER [US] ET AL) 21 December 2017 (2017-12-21) * paragraphs [0055] – [0096]; claims; figures * ----- | 1-16 | |
| X | US 4 427 013 A (NUNN DONALD E [US] ET AL) 24 January 1984 (1984-01-24) * column 5, line 15 – column 9, line 60; claims; figures * ----- | 1,10-16 | |

TECHNICAL FIELDS SEARCHED (IPC)

A61B

The present search report has been drawn up for all claims

| Place of search | Date of completion of the search | Examiner |
|---|---|---|
| The Hague | 25 June 2023 | Crisan, Carmen-Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 15 1041

This annex lists the patent family members relating to the patent documents cited in the above-mentioned European search report.
The members are as contained in the European Patent Office EDP file on
The European Patent Office is in no way liable for these particulars which are merely given for the purpose of information.

25-06-2023

| Patent document cited in search report | | Publication date | Patent family member(s) | | Publication date |
|---|---|---|---|---|---|
| US 2019298188 | A1 | 03-10-2019 | CN | 110301906 A | 08-10-2019 |
| | | | DE | 102019104568 A1 | 02-10-2019 |
| | | | KR | 20190113543 A | 08-10-2019 |
| | | | US | 2019298188 A1 | 03-10-2019 |
| US 2017360313 | A1 | 21-12-2017 | CN | 109310349 A | 05-02-2019 |
| | | | US | 2017360313 A1 | 21-12-2017 |
| | | | WO | 2017222700 A1 | 28-12-2017 |
| US 4427013 | A | 24-01-1984 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2953528 A1

- US 20150320364 A1